# EUROPÄISCHE PATENTSCHRIFT

(11) **EP 2 888 315 B1**
(45) Veröffentlichungstag und Bekanntmachung des Hinweises auf die Patenterteilung: **21.09.2016**
(21) Anmeldenummer: 13783093.1
(22) Anmeldetag: 13.08.2013
(51) Int. Cl.: C08J 3/12, A61L 27/12, A61L 27/24, A61L 27/34, A61L 27/44, A61L 27/52, C08J 3/20, C08L 89/06

(54) **HOMOGENISIERTES KOMPAKTKOMPOSIT, VERFAHREN ZU DESSEN HERSTELLUNG SOWIE KOMPOSITPULVER UND DESSEN VERWENDUNG**
HOMOGENIZED COMPACT COMPOSITE, METHOD FOR PRODUCING SAME, AND COMPOSITE POWDER AND USE THEREOF
COMPOSITE COMPACT HOMOGÉNÉISÉ, PROCÉDÉ POUR LE PRODUIRE, POUDRE COMPOSITE ET UTILISATION DUDIT COMPOSITE COMPACT

(43) Veröffentlichungstag der Anmeldung: 01.07.2015
(73) Patentinhaber: Technische Universität Dresden, 01069 Dresden (DE)
(72) Erfinder: HEINEMANN, Sascha, 01069 Dresden (DE); WORCH, Hartmut, 01187 Dresden (DE)
(74) Vertreter: Kailuweit & Uhlemann Patentanwälte Partnerschaft mbB
(86) Internationale Anmeldenummer: PCT/IB2013/056615
(87) Internationale Veröffentlichungsnummer: WO 2013/190534

(56) Entgegenhaltungen:
- WO-A1-2011/161409
- WO-A2-2010/029150
- HEINEMANN S ET AL: "Bioactive silica-collagen composite xerogels modified by calcium phosphate phases with adjustable mechanical properties for bone replacement", ACTA BIOMATERIALIA, ELSEVIER, AMSTERDAM, NL, Bd. 5, Nr. 6, 1. Juli 2009 (2009-07-01), Seiten 1979-1990, XP026161763, ISSN: 1742-7061, DOI: 10.1016/J.ACTBIO.2009.02.029 [gefunden am 2009-02-28]

## Beschreibung

Die Erfindung betrifft homogenisierte Kompaktkomposite, ein Verfahren zu deren Herstellung und Formgebung sowie deren Verwendung als Implantatmaterial.

Von temporär im Organismus verbleibenden Knochenersatzmaterialien wird gefordert, dass sie einerseits eine ausreichende mechanische Festigkeit haben müssen, also frei von Mikrorissen sind und andererseits einen homogenen Gefügeaufbau aufweisen. Darüber hinaus muss ein solches Material ur- oder umformbar, d.h. in genau vorgegebener Geometrie gestaltbar sein. Diese Forderungen sind verknüpft mit einem definierten Verlauf ihrer Abbaukinetik. Um dem gerecht zu werden, sind spezifische Anforderungen an die Struktur und das Gefüge dieser Materialien zu stellen.

Der natürliche Knochen erreicht diese Eigenschaften durch einen hierarchischen Aufbau unter Mitwirkung der Komponenten Kollagen I und einer Hydroxylapatit kristallografisch ähnlichen Calciumphosphatphase. Die organische Komponente Kollagen I ist zur Selbstorganisation befähigt und bildet Fibrillen mit Hohlraumzonen (gaps), in die Calciumphosphatphase plättchenförmig eingelagert wird. Der Degradationsprozess erfolgt durch Ansäuerung und Herauslösen der Calciumphosphatphase und anschließendem enzymatischen Abbau der organischen Komponente Kollagen. Analog existieren mit den Spikulen der Kieselschwämme Skelettstrukturen, die ebenfalls auf Kollagen als organische Matrix beruhen, jedoch Silikat anstelle von Calciumphosphat als anorganisch-nichtmetallische Komponente aufweisen.

Materialien mit knochenähnlichen Eigenschaften lassen sich in einem biomimetischen Ansatz unter Anwendung einer Kombination der Prinzipien der Säugetierknochen und der Glasschwammspikulen entwickeln.

Die DE 10 2004 058 893 B4 beschreibt mineralisierte modifizierte Biopolymere, bspw. mit Hydroxylapatit kristallisierte Kollagenfibrillen. Diese werden durch die Umsetzung und/oder Lagerung der modifizierten Biopolymeren mit Calcium- und Phosphationen enthaltenden Lösungen gewonnen, wobei die Modifizierung der Biopolymere das Aufwachsen der Kristallite auf dem Kollagen begünstigt.

Aus der DE 10 2005 041 414 A1 sind Glasschwammkollagen und daraus hergestellte Kollagen-Silikat-Verbundmaterialien bekannt. Dabei werden im Zuge einer nicht katalytischen Selbstorganisation Siliziumdioxid-Nanopartikel entlang und auf den Kollagenfibrillen abgeschieden. Die so gewonnenen Proben werden anschließend langsam luftgetrocknet oder lyophilisiert, wobei sich ein kompaktes Material mit erhöhter Festigkeit und Elastizität bildet. Während des Trocknungsprozesses verringert sich das Volumen der gewonnenen Materialien um 40 bis 60%.

In der WO 2008/023025 A1 beschreiben die Erfinder eine Methode, um Hybridmaterialien basierend auf einer silikatisierten Kollagenmatrix herzustellen. Die Grundlage dafür bildet ein Sol-Gel-Prozess, in dem Kieselsäuremoleküle unter bestimmten Bedingungen zu Silikat-Nanopartikeln polymerisieren. Wenn dieser Prozess in Gegenwart von Kollagenfibrillen durchgeführt wird, findet die Polymerisation vorzugsweise an den Fibrillen statt. Bei geeigneter Zusammensetzung lässt sich auf diese Weise ein Hybrid-Hydrogel herstellen. Dieses kann unter geeigneten Bedingungen getrocknet und so in ein Hybrid-Xerogel überführt werden. Da der Feststoffanteil des Hydrogels prozessbedingt jedoch sehr gering ist, lassen sich nur kleine Abmessungen eines monolithischen Materials herstellen. Bei dem Zweistoffsystem Silikat-Kollagen ist somit der Zusammensetzungsbereich, in dem monolithische Xerogele hergestellt werden können, begrenzt. Zudem lässt sich das resultierende Gefüge ausschließlich über die jeweiligen Komponentenanteile des Silikats und des Kollagens beeinflussen. Folglich lassen sich auch die Materialeigenschaften, und dabei vor allem die mechanischen Eigenschaften, nur in einem engen Bereich variieren.

Die WO 2010/029150 A2 offenbart Kompositmaterialien aus einer mit Silikat und Calciumphosphatphasen mineralisierten Kollagenmatrix. Zur Herstellung dieser Materialien wird eine Calciumphosphatphase in einer homogenen Kollagensuspension gelöst und anschließend Kieselsäure in Form eines Silizium-Precursors zugegeben. Gemäß der Schrift erreicht die so erzeugte Mischung ihren Gelpunkt unter Luftabschluss und in einem geeigneten Lösungsmittel. Das Hydrogel wird anschließend unter normalen bzw. überkritischen Bedingungen zu einem Xero- bzw. Aerogel getrocknet. Die geometrische Ausformung der so erhaltenen Gele erfolgt bedingt bereits während der Gelbildung oder nach der Trocknung durch mechanische Bearbeitung, bspw. spanabhebend auf einer konventionellen Drehmaschine. Durch den bei der Trocknung auftretenden Volumenschwund neigen die Xerogele zur Rissbildung, inneren Spannungen und unregelmäßigen, defektbehafteten Gefügen.

Aus der deutschen Patentanmeldung DE 10 2004 012 411 A1 sind Kompositmaterialien auf der Basis von Polykieselsäuren und Verfahren zu deren Herstellung bekannt. Diese enthalten Polykieselsäure, ein organisches Polymer im Anteil von 0,1 bis 20 Masse- %, mindestens eine Calciumphosphatphase mit einem Anteil von mehr als 15 Masse- % und wahlweise ein anwendungsspezifisches Additiv. Um zu den offenbarten Kompositmaterialien zu gelangen, werden die einzelnen Bestandteile des Kompositmaterials in Abhängigkeit von ihren chemischen und physikalischen Eigenschaften aufeinanderfolgend oder kombinationsweise zusammengegeben. Anschließend wird die Mischung homogenisiert, in die gewünschte Form gebracht und dann bei Temperaturen von 100 °C und mehr getrocknet, wobei sich ihr Volumen stark verringert. Die Formgebung erfolgt dabei in Abhängigkeit von der Viskosität des Kompositmaterials, bspw. durch Ausgießen, Auspressen, Spritzen oder Versprühen.

Aus der deutschen Patentanmeldung DE 199 62 090 A1 sind Formkörper in der Geometrie von Knochen oder Knochenteilen bekannt, die aus Kollagen oder mineralisiertem Kollagen in Form eines dichten Netzwerks gebildet sind, die zusätzlich in einer Matrix aus Calciumphosphatzement eingebettet sein können. Die Herstellung der Formkörper erfolgt in einem Gefriertrocknungsprozess. Dazu wird eine Suspension mineralisierten Kollagens mit der Oberfläche einer gekühlten Form in Kontakt gebracht und durch Gefrieren auf dieser abgeschieden. Dieser Vorgang wird ein oder mehrmals wiederholt, wobei mittels Verwendung verschieden konzentrierter Suspensionen auch Formkörper mit Dichtegradienten herstellbar sind.

Die deutsche Patentanmeldung DE 10 2006 014 522 A1 offenbart ein Verfahren zur Herstellung eines anorganischen Knochenersatzmaterials, bei dem ein als Lösungsmittel Wasser und/oder Alkohol aufweisendes Sol einer oxidischen Verbindung mit darin homogen verteilten Calciumphosphatphasen in eine unterhalb des Gefrierpunktes des verwendeten Lösungsmittels abgekühlte Gefrierform gegossen wird. Das Lösungsmittel wird unter Gelbildung gefroren und anschließend das gefrorene Lösungsmittel durch Gefriertrocknung entzogen, wodurch ein offenporiges Gelstützgerüst mit den eingebetteten Calciumphosphatphasen entsteht. Die Porosität des entstehenden Knochenersatzmaterials wird durch den Wasseranteil des Sols sowie die Prozessführung, insbesondere die Abkühlgeschwindigkeit, gesteuert. Dabei hat insbesondere die Gefriertrocknung einen wesentlichen Einfluss auf die Struktur des Knochenersatzmaterials.

In der deutschen Patentanmeldung DE 198 11 900 C2 wird ein biokompatibles Kompositmaterial beschrieben, das ein anorganisches Gel und als bioaktive Komponente ein oder mehrere homogen eingebettete Skleroproteine oder deren Hydrolyseprodukte und/oder Glykosaminoglykane enthält. Durch Zusatz von Calciumsalzen, Phosphaten oder basischen Calciumphosphat-Suspensionen kann die Bioaktivität des Komposits erhöht werden.

Die US 7,718,616 B2 beschreibt eine biokompatible Zusammensetzung mit Partikeln aus einer Kollagen- und einer Calciumphosphatkomponente und einer bevorzugten Größe zwischen 100 µm und 840 µm. Diese Partikel sind mit einer Flüssigkeit, bspw. Blut oder Blutserum, zu einer Paste mischbar und können so auf einen zu behandelnden Knochendefekt appliziert und dort vollständig resorbiert werden. Zu Transportzwecken lassen sich die Partikel in scheibenförmigen Presslingen verarbeiten, die sich in Flüssigkeiten lösen und mit diesen mischbar sind.

Mit der DE 10 2009 060 623 A1 wird ein künstlicher Knochen offenbart, der durch Überlappen und Aufrollen zumindest eines Apatit/Kollagen-Verbundstoffblatt mit einem Kollagenblatt erzeugt wird. Dadurch entsteht ein künstlicher Knochen mit hohlem zylindrischen Mittelteil und einem Durchmesser von 100-1000 µm. Die konstituierenden Apatit/Kollagen- bzw. Kollagen-Blätter werden auf konventionelle Weise über Sol-Gel-Verfahren und Flächenpressung erzeugt.

Die US 6,478,825 B1 beschreibt ein Knochenimplantat, dass aus zerkleinerten natürlichen Knochenelementen besteht, die nach der Zerkleinerung demineralisiert, in eine gewünschte Form gefügt und erneut mit einem mineralischen Bindemittel verfestigt werden.

In der US 2006/02658062 A1 wird ein resorbierbarer, biokompatibler und geformter Körper beschrieben, der als organische Komponente Gelatine und als anorganische Komponente Calciumphosphatverbindungen aufweist und aus Nanopartikeln mit einer Partikelgröße zwischen 7 und 800 nm gebildet ist. Die organische Komponente ist innerhalb von 3 bis 20 Tagen vom Körper resorbierbar, wobei die Resorbierbarkeit durch Hydrophobierung verringert werden kann. Die anorganische Komponente ist innerhalb von 7 bis 180 Tagen resorbierbar, wobei die Resorbierbarkeit der anorganischen Komponente durch kleine Partikelgrößen erhöht werden kann.

Ein Verfahren zur Herstellung mineralisierter Kollagenfibrillen wird auch in der US 5,455,231 A beschrieben. Einer Lösung entmineralisierten Kollagens werden demnach bei einem pH Wert zwischen 10 und 13 lösliche Calcium- und Phosphatverbindungen zugegeben. Mineralisiertes Kollagen wird anschließend gesammelt und zu einem Pulver gefriergetrocknet. Nanoskopische Hydroxylapatit-Partikel mit einer Größe zwischen 1 und 9 nm sind auch in der US 2011/0008460 A1 offenbart. In einer bevorzugten Ausführungsform des Verfahrens werden diese Partikel mit Kollagen als Matrixmaterial vermengt.

Die CN 1 338 315 A offenbart eine *"nm-crystal collagen-based calcium phosphat composition"* sowie Schritte zu deren Herstellung. Mit der DE 10 2004 012 411 A1 sind Kompositmaterialien auf der Basis von Polykieselsäuren und Verfahren zu deren Herstellung offenbart. Diese enthalten Polykieselsäure, ein organisches Polymer im Anteil von 0,1 bis 20 m-% sowie mindestens eine Calciumphosphatphase mit einem Anteil von mehr als 15 m-%. Gemäß der Schrift kann das Material auch in Form eines Granulates oder Pulvers vorliegen.

Die oben genannten Dokumente beschreiben vor allem monolithische Kompositxerogele, die aus Hydrogelen hergestellt werden. Sollen diese direkt als Implantat oder als Rohling für eine mechanische Bearbeitung zur Herstellung eines Implantats genutzt werden, so offenbaren sich viele Nachteile.

Da mit der Umwandlung vom Hydrogel zum Xerogel eine Strukturänderung und damit Volumenkontraktion auf etwa 10-30% des Ausgangsvolumens einhergeht, entstehen im Gel Mikro- und Makrodefekte, vorwiegend in Form von Rissen und Hohlräumen. Die Anzahl, Form und Art der Defekte ist dabei kaum vorhersagbar und von außen nicht zu charakterisieren. Diese Heterogenität des Gefüges hat zur Folge, dass ein entsprechendes Implantat unter Last unerwartet versagen kann.

Aufgrund der Volumenkontraktion bei der Trocknung der Gele und der damit verbundenen Strukturänderung weisen die erhaltenen Xerogelrohlinge eine sehr unregelmäßige Geometrie auf. Dies macht, insbesondere bei komplexeren Implantatgeometrien eine nachträgliche maschinelle Bearbeitung in den meisten Fällen unerlässlich. Die Schaffung einer definierten Geometrie wird dabei jedoch häufig durch die unregelmäßige innere Struktur der Rohlinge erschwert, insbesondere, wenn durch die Volumenkontraktion Spannungen im Material entstanden sind.

Die starke Volumenkontraktion auf etwa 10-30% des Ausgangsvolumens beim Übergang vom Hydrogel zum Xerogel führt zudem zu einer drastischen Beschränkung der erzielbaren Maximalgröße der Implantate bzw. Rohlinge. Da sich mit heute bekannten Sol-Gel-Verfahren Hydrogele nur mit einem Volumen von max. etwa 10 - 20 cm³ homogen herstellen lassen, ist die Maximalgröße der so erzeugten Implantatrohlinge ebenfalls auf wenige Kubikzentimeter beschränkt.

Zum Teil werden die mit der Sol-Gel-Methode erhaltenen monolithischen Xerogele daher nur als Vorstufe zur Implantatherstellung eingesetzt. Dann werden die erhaltenen Gele im Anschluss an die Trocknung gemahlen, gepresst und thermisch behandelt. Bei hohen Temperaturen von meist mehreren hundert Grad Celsius wird das Material danach wieder zu einem Monolith gesintert, wobei jedoch die organischen Bestandteile des Materials zerstört werden, die in der Regel nicht über den Siedepunkt von Wasser hinaus temperaturbeständig sind.

Weiterhin bekannt ist die Vermengung der gemahlenen Xerogele mit Flüssigkeiten sowie Bindemittel um Knochenzemente herzustellen, die direkt auf Knochendefekte appliziert werden können. Auch dabei stellt sich das Problem des Volumenschwunds und der damit verbundenen Formuntreue.

Die Aufgabe der Erfindung ist es daher, ein Verfahren sowie Stoffsysteme zur Herstellung von Organik/Anorganik-Kompositenmaterialien mit homogenem und defektarmen Gefüge bereitzustellen, insbesondere für Biokompositmaterialien mit knochenähnlichen Eigenschaften. Die Herstellung der Kompositmaterialien soll dabei energie-, kosten- und materialschonend erfolgen und eine Einstellung der mechanischen Eigenschaften der Kompositmaterialien ermöglichen. Insbesondere soll die Funktionalität der organischen Phase, in biomimetischer Weise eine Verbindung mit der anorganischen Phase einzugehen, aufrechterhalten werden. Weiterhin soll mit dem Verfahren die Herstellung beliebig geformter sowie ausgedehnter Kompositkörper ermöglicht werden.

Erfindungsgemäß wird die Aufgabe gelöst durch ein Verfahren zur Herstellung eines homogenisierten Kompaktkomposits aus unregelmäßig mit Silikat mineralisiertem Kollagen mit den Verfahrensschritten:
a) Erzeugen eines Hydrogels durch Mischen eines Silizium-Precursors und einer Kollagensuspension,
b) Trocknung des Hydrogels zu einem Kompositmaterial aus einer mit Silikat mineralisierten Kollagenmatrix in Form eines Xero- oder Aerogels,
c) Zermahlen eines Kompositmaterials aus einer mit Silikat mineralisierten Kollagenmatrix zu einem Kompositpulver, enthaltend Partikel mit einer definierten Partikelgrößenverteilung, insbesondere einem Äquivalentdurchmesser kleiner 0,700 mm, aus einzelnen oder als Agglomerat vorliegenden, unregelmäßig mit Silikat mineralisiertem Kollagen,
d) Verpressen der Kompositpulver bei Temperaturen unter 40°C und Pressdrücken von 700MPa bis zu 1 GPa zu einem formstabilen homogenisierten Kompaktkomposit mit defektarmen Gefüge.

Ausgangsstoff des erfindungsgemäßen Verfahrens zur Herstellung eines homogenisierten Kompaktkomposits sind Kompositmaterialien aus einer mit Silikatmineralisierten Kollagenmatrix. Diese wird dazu nasschemisch, z. B. mittels Sol-Gel-Technik, mit einer anorganischen Phase, insbesondere einem Silikat mineralisiert. Das Kollagen, bzw. die von diesem gebildeten selbstorganisierten Strukturen in Form hochvernetzter, komplexer Netzwerke, ist somit der Keimbildner für die Kristallisation der anorganischen Phase. Zusätzlich kann das Kollagen sowohl hinsichtlich der Faser- bzw. Netzwerkbildung, als auch hinsichtlich der Kristallisation der anorganischen Phase, biochemisch modifiziert sein. Insbesondere können dadurch eine große spezifische Oberfläche des Kollagens und/oder optimale Bindungsverhältnisse zwischen beiden Phasen erzeugt werden.

Als Kollagenkomponente der silikatisierten Kollagenmatrix kann jegliches Kollagen eingesetzt werden, d.h. Kollagen jeglicher Form (z. B. fibrilläres Kollagen, netzbildendes Kollagen, faserbildendes Kollagen, Tropokollagen, teilweise oder vollständig fibrilliertes Kollagen, teilweise oder vollständig (irreversibel) denaturiertes Kollagen, Kollagenfasern oder größere Aggregate usw.) und jeglichen Typs (z. B. Typ I, II, III, IV, V, VI, VII, VIII, IX, X, XI, XII, XIII, XIV, XV, XVI, XVII, XVIII, XIX, XX, XXI, XXII, XXIII, XXIV, XXV, XXVI).

Die Kollagenkomponente des erfindungsgemäßen Kompaktkomposits kann dabei rekombinantes Kollagen, Kollagen aus Eumetazoa (d.h. Gewebetiere, darunter Nesseltiere und Bilateria), Schwammkollagen der Klassen Demospongia (Hornschwämme) oder Calcarea (Kalkschwämme), ein synthetisches Kollagenanalogon, ein Kollagenderivat oder eine Mischung dieser Kollagene sein.

Kollagenanaloga sind synthetisch hergestellte Polypeptidketten, deren Primärsequenz so gestaltet ist, dass sie bestimmte Eigenschaften der nativen Kollagenmonomere simulieren können, z. B. Tripelhelixbildung, Fibrillogenese, Gelbildung. Rekombinante Kollagene sind solche, deren Primärsequenz identisch ist mit der von Kollagen Typ I. Sie werden mithilfe genetisch manipulierter Mikroorganismen hergestellt und gegebenenfalls nachbehandelt. Kollagenderivate sind Verbindungen, die sich von der Grundverbindung Kollagen formal ableiten und aus ihr herstellen lassen.

Vorteilhaft ist das eingesetzte Kollagen zusätzlich biochemisch modifiziert. Die Modifikation der Kollagenkomponente beruht dabei auf einer gezielten Beeinflussung der Fibrillengeometrie (Durchmesser und Länge) durch biochemische Eingriffe in die Fibrillogenese. Da die Fibrillen im Kompositpulver und im Kompaktkomposit die Funktion einer Faserverstärkung einnehmen, hat ihre Geometrie Einfluss auf die mechanischen Eigenschaften der Verbundmaterialien.

Mithilfe von Aminozucker-haltigen Polysacchariden, bevorzugt von Glykosaminoglykanen und deren Analoga, lässt sich die Fibrillengeometrie vorteilhaft gezielt beeinflussen. Zu Glykosaminoglykanen analoge Verbindungen sind chemische Verbindungen mit gleicher biologischer Wirkung.

Besonders bevorzugt ist dabei die biochemische Modifikation des Kollagens durch die Glykosaminoglykane Chondroitinsulfat, Dermatansulfat, Heparin, Heparansulfat, Hyaluronsäure und Keratansulfat, aber auch durch Proteoglykane, wie Decorin, und andere Aminozucker-haltige Polysaccharide wie Chitosan. [Wolf C., Hanke T., Scharnweber D., Peters K., Kirkpatrick C. J., Worch H.: Influence of artificial extracellular matrices on Ti6AI4V implants on binding and release of VEGF, Abstracts Internationales Symposium ,Interface Biology of Implants', Rostock 14.-16.05.2003, in: BlOmaterialien 4: 158, 2003; Wolf-Brandstetter C., Lode A., Hanke T., Scharnweber D., Worch H.: "Influence of Modified Extracellular Matrices on Ti6AI4V implants on binding and release of VEGF". J. Biomed. Mat. Res A. 79: 882-894, 2006; Abschlussbericht "Innovative Materialsysteme für die Funktionalisierung von Implantaten und den Gewebeaufbau in der Implantologie" (Autoren: Worch, H., Scharnweber, D., Hanke, T., 2002) BMBF-Projekt 03N4015/9 (10/1999 bis 10/2002); Sammlung "Deutsche Forschungsberichte" TIB/UB Hannover].

Die biochemische Modifikation erfolgt durch den Einbau der genannten Substanzen in die Kollagenmatrix. Der Einbau erfolgt bevorzugt entweder durch adsorptive Immobilisierung, oder durch kovalente Quervernetzung, beispielsweise mit N-(3-dimethylaminopropyl)-N'-ethylcarbodiimide (EDC). Der Einbau kann alternativ auch während der spontanen Zusammenlagerung der Fibrillen stattfinden, die bei neutralem pH-Wert (z. B. in Phosphatpuffer oder Tris-Puffer) und auf ca. 37°C erhöhter Temperatur erreicht wird. Im Anschluss daran erfolgt dann gegebenenfalls eine kovalente Quervernetzung.

Die organische Komponente des erfindungsgemäßen Kompaktkomposits können somit auch vorstrukturierte Kollagentemplate, vorstrukturierte denaturierte Kollagene, vorstrukturierte Kollagenmoleküle, vorstrukturierte Kollagenmikrofibrillen, vorstrukturierte Kollagenanaloga und/oder vorstrukturierte Kollagenfragmente mit einer Länge von vorzugsweise 50 bis 500 nm sein. Dabei bezeichnet Vorstrukturierung im Sinne der Erfindung, dass die vorstrukturierten Kollagentemplate biochemisch modifiziert bzw. so funktionalisiert sind, dass an deren Oberfläche Hydroxylapatit aufwachsen kann, der aufgrund der Größe, Vorstrukturierung und Diffusions- und Migrationsfähigkeit der vorstrukturierten Kollagentemplate wachstumsinhibiert wird. Die Funktionalisierung kann natürlichen Ursprungs sein oder durch Vorstrukturierung mit Zuckermolekülen oder nichtkollagenen Proteinen erreicht werden. Für eine detaillierte Beschreibung der Erzeugung und Wirkung wachstumsinhibierten Hydroxylapatits wird auf die DE 10 2011 052 416 A1 verwiesen.

Bei den Silikaten handelt es sich vorzugsweise um Sol-Gel-Silikate, hergestellt aus Silanen die zunächst hydrolisiert werden. Als Zwischenprodukt entsteht dabei Kieselsäure, die wiederum zu Silikat polymerisiert. Bevorzugt handelt es sich bei der Silikat-Komponente um eine aus einer eine pre-hydrolisiertes Alkoxysilan-Lösung abgeschiedene Phase, besonders bevorzugt um eine aus einer Tetramethoxysilan-(TMOS)-Lösung, Tetraethoxysilan-(TEOS)-Lösung oder Wasserglas abgeschiedenen Phase.

Bevorzugt erfolgt die Herstellung des Ausgangskompositmaterials indem zunächst ein Silizium-Precursor, in einer homogenen Kollagensuspension gelöst und so ein Hydrogel erzeugt wird. Als Siliziumprecursor wird dabei bevorzugt eine hydrolisierte Alkoxysilan-Lösung, bspw. Tetramethoxysilan-(TMOS)-Lösung oder Tetraethoxysilan-(TEOS)-Lösung, oder Wasserglas eingesetzt. Nach Erreichen des Gelpunkts wird das Hydrogel, zur Festigung der Festkörperstruktur, unter Luftabschluss und in einem geeigneten Lösungsmittel gelagert.

Die im Sol-Gel Prozess erzeugten Hydrogele werden anschließend entweder zu einem Xerogel getrocknet oder nach Substitution der Flüssigphase des Hydrogels unter überkritischen Bedingungen zu einem Aerogel getrocknet. Dadurch wird ein Kompositmaterial aus einer mit Silikat mineralisierten Kollagenmatrix und mit hoher Bioaktivität gewonnen, das hinsichtlich der Umformbarkeit, Verarbeitbarkeit, Struktur und Textur die in der Würdigung des Standes der Technik genannten Nachteile aufweist.

Für eine detaillierte Beschreibung des Ablaufs des Sol-Gel-Prozesses und der anschließenden Trocknung sowie von prozessualen Einstellmöglichkeiten und deren Auswirkung auf die entstehenden Gele sei auf die WO 2008/023025 A1 bzw. die DE 10 2008 047 405 A1 oder US 2011/0237552 A1 verwiesen, auf die hier und im Folgenden Bezug genommen wird. Zur oben genannten biochemischen Modifizierung sei im Detail auf die DE 10 2004 058 893 A1 verwiesen.

Vorteilhaft können bereits bei der Herstellung der mineralisierten Kollagenmatrix pharmazeutische Wirkstoffe integriert werden.

### Verfahrensschritt a)

Erfindungsgemäß wird das Ausgangskompositmaterial zur Überwindung der oben genannten Nachteile im erfindungsgemäßen Verfahren zu einem Kompositpulver definierter Partikelgrößenverteilung und einem Äquivalentdurchmesser kleiner 0,700 mm zermahlen. Dabei wird die mineralisierte Kollagenmatrix aufgebrochen und zerkleinert, der Verbund zwischen Kollagen und anorganischer Phase bleibt jedoch bestehen. Das entstehende Pulver besteht somit aus einzeln oder als Agglomerat vorliegendem, unregelmäßig mit Silikat mineralisiertem Kollagen, wobei die einzelnen Pulverpartikel die Substruktur der ursprünglichen Kollagenmatrix aufweisen. Die organische Phase bzw. das Kollagen kann dabei stellenweise vollständig von den darauf plättchenförmig abgelegten anorganischen Kristalliten bedeckt sein, an anderer Stelle jedoch unbedeckt zu Tage treten.

Das Zermahlen des Kompositmaterials findet derart statt, dass die entstehenden Partikel eine definierte Partikelgrößenverteilung, insbesondere einen Äquivalentdurchmesser, bevorzugt einen volumenäquivalenten Kugeldurchmesser, von weniger als 0,700 mm, bevorzugt zwischen 0,001 und 0,700 mm, besonders bevorzugt zwischen 0,05 und 0,700 aufweisen. Dadurch weisen die Partikel vorzugsweise eine sehr große spezifische Oberfläche im Verhältnis zu Ihrem Volumen auf. Daraus resultiert eine hohe Reaktivität der Teilchen, bedingt sowohl durch die anorganischen Phasen, als auch hinsichtlich des zum Teil an der Oberfläche der Partikel vorliegenden Kollagens.

Der Zermahlvorgang selbst läuft dabei trocken ohne die Zugabe einer zusätzlichen flüssigen Phase ab. Dabei wird das Ausgangskompositmaterial mit einer geeigneten Mühle, bevorzugt einer Kugelmühle, Planetenmühle, Trommelmühle, Wirbelstrommühle, Vertikalmühle, Rotormühle, Rohrmühle oder einer Asima-Mühle mechanisch zermahlen. Dabei wird die Temperatur des Mahlguts, gegebenenfalls durch eine zusätzliche Kühlung unter 40°C gehalten, um so die Struktur und die biologische Funktionalität des Kollagens zu erhalten und dessen Denaturierung zu vermeiden. Ebenfalls bevorzugt ist das Zermahlen des Kompositmaterials mittels einer Luftstrahlmühle.

Kann die Partikelgrößenverteilung im Zermahlvorgang nicht genau genug eingestellt werden, erfolgt anschließend vorzugsweise eine Siebung des Pulvers. Bevorzugt erfolgt dies in einer Trockensiebung, wobei das Pulvermaterial in einem Siebsystem nacheinander mittels mehrerer Siebe mit kleiner werdender Maschenweite gesiebt wird. Dadurch können gezielt verschiedene Fraktionen des Pulvers mit bestimmter Partikelgrößenverteilung, insbesondere mit einem bestimmten Äquivalentdurchmesser gewonnen werden. Die Siebung kann gleichzeitig als Siebanalyse zur Bestimmung der Partikelgrößenverteilung des Pulvermaterials bzw. der Äquivalentdurchmesser, der Pulverpartikel genutzt werden. Somit kann die Partikelgrößenverteilung des gewonnen Pulvers bzw. der Anteil von Partikeln eines bestimmten Äquivalentdurchmessers, bevorzugt eines volumenäquivalenten Kugeldurchmessers, bestimmt werden. Mit Hinblick auf die Genauigkeit von üblichen Siebverfahren, beziehen sich die Angaben zum Äquivalentdurchmesser der Partikel auf mindestens 75 Masse-%, bevorzugt mindestens 85 Masse-% und besonders bevorzugt mindestens 95 Masse-% des Pulvers.

Das Kompositmaterial wird bevorzugt zu einem Kompositpulver mit einer Schüttdichte von mehr als 0,1 g/cm³, vorzugsweise von zwischen 0,2 und 0,4 g/cm³ und besonders bevorzugt von mehr als 0,5 g/cm³ zermahlen. Dadurch weist das Pulver eine gute Fließfähigkeit auf und ist dadurch in den weiteren Schritten des erfindungsgemäßen Verfahrens vorteilhaft optimal verarbeitbar. Die Schüttdichte ist dabei auch von der Partikelgrößenverteilung des Kompositpulvers, insbesondere von dem Äquivalentdurchmesser der Pulverpartikel, abhängig.

### Verfahrensschritt b)

Erfindungsgemäß wird das durch Zermahlen und ggf. Sieben erhaltene Kompositpulver anschließend bei Raumtemperatur und vergleichsweise geringen Pressdrücken von 700 MPa bis 1 GPa, bevorzugt zwischen 700 und 900 MPa, zu einem formstabilen Festkörper bzw. einem homogenen Kompaktkomposit verpresst. Diese sind formstabil und weisen auch ohne die Zugabe von zusätzlichem Bindemittel eine hohe Festigkeit und ein kompaktes Gefüge auf. Durch die Presswerkzeuge wird dabei die Geometrie der monolithischen Formkörper vorgegeben, die ansonsten hinsichtlich der Urformbarkeit keinen Beschränkungen unterliegen. Die erzeugten homogenen Kompaktkomposite weisen vorteilhaft ein defektarmes Gefüge auf, das insbesondere frei von Makrodefekten und Mikrodefekten wie Hohlräumen, Poren, Rissen und Mikrorissen ist. Solche Defekte entstehen unvermeidbar bei der Herstellung der Hydrogele durch Lufteinschlüsse beim Mischvorgang sowie insbesondere bei der Überführung der Hydrogele in Xerogele aufgrund der dabei auftretenden Kapillarspannungen und Eigenspannungen. Diese im Ausgangsmaterial vorhandenen Defekte werden zum einen durch den Mahlvorgang aufgeschlossen und freigelegt. Zuvor innere Oberflächen der monolithischen Xerogele werden dadurch zu äußeren Oberflächen der Pulverpartikel. Zum Zweiten werden im Rahmen des Pressvorganges mögliche Defekte in den Pulverpartikeln selbst verdichtet. Es verbleiben lediglich Korngrenzen zwischen den einzelnen Pulverpartikeln.

Aufgrund der bevorzugten Partikelgrößenverteilung bzw. Schüttdichte des Kompositpulvers ist dieses gut fließfähig und wird dadurch beim Verpressen vorteilhaft homogen kompaktiert. Dadurch kann die Entstehung von Inhomogenitäten der Dichte bzw. des Gefüges des entstehenden Kompaktkomposits vorteilhaft verhindert werden. Unter einem homogenen Gefüge ist im Sinne dieser Patentanmeldung zu verstehen, dass die scheinbare Dichte des Kompaktkomposits über ein jeweils untersuchtes Volumen konstant ist. Die scheinbare Dichte des Kompaktkomposits variiert somit innerhalb eines Volumens von 1 mm³, bevorzugt in einem Volumen vom 10 mm³ und besonders bevorzugt in einem Volumen von 500 bis 1000 cm³ lediglich minimal, bevorzugt nicht mehr als um 0,1 g/cm³, bevorzugt nicht mehr als um 0,05 g/cm³ und besonders bevorzugt nicht mehr als um 0,01 g/cm³. Die scheinbare Dichte bzw. Rohdichte bezeichnet dabei die Dichte des porösen Körpers inklusive der Poren. Die scheinbare Dichte ist somit lediglich bei einem unporösen Körper gleich der Reindichte. Neben den Poren sind in dem homogen kompaktierten Material keine weiteren, insbesondere inhomogen verteilten, Hohlräume vorhanden. Beim Verpressen wird ebenfalls darauf geachtet, dass die Temperatur im Pressgut 40°C nicht überschreitet, damit das Kollagen vorteilhaft seine Struktur bzw. seine biologische Funktionalität bewahrt.

Bei der Kompaktierung ist man vorteilhaft nicht auf Pulvermaterial beschränkt, das zuvor aus einem einzelnen monolithischen Xero- oder Aerogel gemahlen wurde. Durch die Mischung verschiedener Pulveransätze gleicher oder verschiedener Zusammensetzung lässt sich vielmehr chargenübergreifend kompaktieren, wodurch beliebig ausgedehnte Formkörper herstellbar sind. Zudem erlaubt die Mischung von Kompositpulver verschiedener Herkunft eine Homogenisierung der Zusammensetzung bzw. deren gezielte Einstellung durch Kombinationen verschiedener Pulver.

Bevorzugt werden zur Mineralisation der organischen Phase Silikat und Calciumphosphatphasen eingesetzt. Dabei sind alle Calciumphosphatverbindungen einsetzbar, die eine homogene Mischung mit der Silikatlösung und der Kollagensuspension erlauben. Vorzugsweise finden dabei solche Calciumphosphatphasen Verwendung, die in *vivo* in Hydroxylapatit umgewandelt werden. In einer bevorzugten Ausführungsform wird als Calciumphosphatphase Hydroxylapatit (Ca₁₀(PO₄)₆(OH)₂), Tricalciumphosphat (Ca₃PO₄), amorphes Calciumphosphat (Caₓ(PO₄)_{y}·nH₂O), Octacalciumphosphat (Ca₈H₂(PO₄)₆·5H₂O) oder Brushit (CaHPO₄·2H₂O) oder Mischungen dieser Calciumphosphatphasen untereinander, wie beispielsweise Calciumphosphatzement (eine Mischung aus Ca₃(PO₄)₂, CaCO₃, Ca(HCO₃) und Ca₁₀(PO₄)₆(OH)₂), oder Mischungen von bzw. mit Calcium- und Phosphatsalzen wie beispielsweise CaCl₂ und/oder Na₂HPO₄ genutzt. Die Calciumphosphatphase kann dabei zusätzlich mit Ionen wie Fluorid, Silber, Magnesium oder Carbonat dotiert sein.

In einer bevorzugten Ausführungsform wird als Calciumphosphatphase nanoskopischer Hydroxylapatit (Ca₁₀(PO₄)₆(OH)₂) eingesetzt, da sich dieser besonders homogen einbringen lässt. Eine homogene Verteilung der Calciumphosphatphase im Material bewirkt besonders gut reproduzierbare Materialeigenschaften. Nanoskopischer Hydroxylapatit besteht im Wesentlichen aus Hydroxylapatit- Nanopartikeln, die vorzugsweise eine Größe von 10 bis 500 nm, bevorzugt eine Größe von weniger als 100 nm aufweisen.

Nach einer weiteren bevorzugten Ausführungsform wird als Calciumphosphatphase wachstumsinhibierter Hydroxylapatit eingesetzt, wie er in DE 10 2011 052 416 A1 offenbart ist. Der wachstumsinhibierte Hydroxylapatit ist in Agglomeraten von vorstrukturierten Kollagentemplaten enthalten, an denen epitaktisch Hydroxylapatitkristallite mit einer Kristallitgröße unterhalb ihres kritischen Keimradius formiert sind. Hergestellt wird er mit den Schritten
a) Mineralisation von vorstrukturierten Kollagentemplaten in übersättigten Ca- und Phosphationen aufweisenden Lösungen, wobei die vorstrukturierten Kollagentemplate diffusions- bzw. migrationsfähig sind, so dass eptaktisch HAP-Kristallite an den Kollagentemplaten aufwachsen und sich die mit HAP-Kristalliten aufgewachsenen Kollagentemplate aufgrund deren Diffusions- bzw. Migrationsfähigkeit agglomerieren,
b) Abtrennen der Agglomerate.

Bei Verwendung von wachstumsinhibiertem Hydroxylapatit können vorteilhaft eine erhöhte Degradabilität des Implantats sowie eine vermehrte *in situ* Freisetzung von Calcium- und Phosphationen erreicht werden. Beides wirkt sich positiv auf die Einheilung des Implantats sowie die Umwandlung des Implantatmaterials in gesundes Gewebe aus.

Die vorteilhafte Kompaktierbarkeit bzw. Verpressbarkeit des Kompositpulvers zu einem homogenen Kompaktkomposit mit defektarmen Gefüge, wurde überraschenderweise gefunden und ist aus dem Stand der Technik nicht bekannt. Es wird vermutet, dass sich anorganische Phasen wie z. B. Silikat und/oder Calciumphosphatphasen zu einem Formkörper vorgegebener definierter Gestalt kompaktieren lassen, wenn ihre molekularen bzw. ionaren Bausteine zuvor an organischen Phasen, wie z. B. Kollagenfibrillen, gebunden wurden und aus dem so entstandenen, nah- oder ferngeordneten Verbund Partikel erzeugt werden.

Dabei setzt die Kompaktierbarkeit voraus, dass das in einem nass-chemischen Verfahren erzeugte monolithische Kompositmaterial zunächst zu Pulver mit einer definierten Partikelgrößenverteilung undmit einem Äquivalentdurchmesser kleiner 0,700mm zermahlen wird. Erst dieses Pulver weist dann die zur Herstellung von stabilen Formkörpern notwendige Kompaktierbarkeit auf.

Weder einphasige Kollagene noch einphasige Silikate, Calciumphosphatphasen oder anorganische Mischungen allein lassen sich bei Raumtemperatur ohne zusätzliche Bindemittel zu Festkörpern mit dem gewünschten Eigenschaftsprofil verarbeiten. Insbesondere lassen sie sich nicht durch Verpressen zu Monolithen kompaktieren.

Der Zusammenhalt des Presslings auch ohne zusätzliche Bindemittel und die vorteilhaften mechanischen Eigenschaften desselben stellen somit ein weiteres überraschendes Ergebnis der Erfindung dar. Die Verbesserung der mechanischen Eigenschaften wird vor allem durch die Beseitigung bzw. Verringerung von Gefügedefekten und inneren Spannungen sowie durch die Erhöhung der scheinbaren Dichte der Presslinge gegenüber den ursprünglichen Xerogelen erreicht.

Bevorzugt ist die scheinbare Dichte des homogenisierten Kompaktkomposits in Abhängigkeit des Drucks beim Verpressen des Kompositpulvers im Bereich von 0,5-5 g/cm³, bevorzugt von 1-3 g/cm³ und besonders bevorzugt von 1,5-2,5 g/cm³ einstellbar.

Diese Einstellbarkeit der Dichte erlaubt vorteilhaft eine Herstellung von Knochenersatzmaterialien, mit denen sich die Eigenschaften vieler verschiedener Knochentypen nachahmen lassen. Im erfindungsgemäßen Verfahren werden bevorzugt mechanische oder hydraulische Presswerkzeuge verwendet, mit denen eine Presskraft von weniger als 10 kN, bevorzugt zwischen 10 und 50 kN und besonders bevorzugt von bis zu 100 kN auf das Kompositpulver ausgeübt werden kann. Die dabei auf die Probenkörper übertragenen Pressdrücke bzw. Spannungen hängen dann zusätzlich von der Größe der in den Presswerkzeugen verwendeten Pressplatten als auch von der Aufstandsfläche des Probenkörpers, bzw. der Größe der Wechselwirkungsfläche zwischen diesen beiden ab. Aufgrund der Abhängigkeit des Pressdrucks von Presskraft sowie der Größe der Wechselwirkungsfläche wird vorteilhaft ein Presswerkzeug bzw. eine Pressvorrichtung beinhaltend ein Presswerkzeug verwendet, das die Einstellung einer Zieldichte des homogenisierten Kompaktkomposits erlaubt. Das Verpressen des Kompositpulvers erfolgt dann so, dass das homogenisierte Kompaktkomposit auf die eingestellte Zieldichte verdichtet ist.

Neben den mechanischen Möglichkeiten, auf die Dichte der Kompaktkomposite Einfluss zu nehmen, kann durch Zugabe von Porogenen zum Kompositpulver und deren Lösung aus dem Gefüge des Kompaktkomposits dessen Porosität gezielt erhöht werden. Damit lässt sich die ursprünglich mesoporöse Porengrößenverteilung im Bereich von 2 nm bis 50 nm zu höheren Wertenim Bereich von 50 bis 200 µm, besonders bevorzugt von 100 bis 500 µm verschieben.

Bei dieser Porosierung wird dem Zwischenprodukt des erfindungsgemäßen Verfahrens, dem Kompositpulver eine lösliche Substanz, zum Beispiel Natriumchlorid, physikalisch beigemischt. Die Menge der beigemischten Substanz ist dabei in Hinblick auf die Kompaktierbarkeit des Pulvermaterials zu beschränken. Nach dem Verpressen des Pulvermaterials zum Kompaktkomposit wird die beigemischte Substanz auf geeignete Weise, bspw. durch Spülung mit einem Lösungsmittel, wieder aus dem Gefüge des Kompaktkomposits gelöst. Dadurch kann das Hohlraumvolumen des Kompaktkomposits auch im Anschluss an den Pressvorgang erhöht und dessen scheinbare Dichte erniedrigt werden. Besonders vorteilhaft kann so ein poröses aber dennoch hochfestes Kompaktkomposit erzeugt werden, dessen scheinbare Dichte geringer ist als dessen Reindichte.

Als Porogene werden bevorzugt anorganische Salze, besonders bevorzugt Natriumchlorid, Zuckerkristalle oder Paraffin- oder Gelatinepartikel genutzt. Insbesondere werden nur solche Stoffe als Porogene genutzt, die während des Pressens des Kompositpulvers ihre Form zumindest weitgehend erhalten und nach dem Verpressen durch Zugabe eines geeigneten Lösungsmittels relativ einfach aus dem Gefüge des Kompaktkomposit lösbar sind.

Weiterhin bevorzugt werden dem Kompositpulver vorteilhaft pharmazeutische Wirkstoffe und/oder ein Bindemittel zugesetzt.

Diese Stoffe können dabei schon im nass-chemischen Verfahren in das Kompositmaterial integriert und so Bestandteil des Kompositpulvers geworden sein. Dabei unterliegen die beimischbaren Konzentrationen jedoch starken Beschränkungen, da die Bildung von stabilen Hydrogelen nur innerhalb bestimmter Konzentrationsbereiche der organischen und anorganischen Phase möglich ist und der Feststoffanteil von Hydrogelen nicht beliebig erhöht werden kann. Die Möglichkeit der Beimischung nicht-löslicher Substanzen ist im nass-chemischen Verfahren ebenfalls nicht gegeben. Im erfindungsgemäßen Verfahren hingegen können vorteilhaft sowohl höhere Substanzkonzentrationen als auch nicht-lösliche Stoffe in das Kompaktkomposit eingebracht werden, indem man diese dem Kompositpulver physikalisch beimischt. Mengenmäßige Einschränkungen ergeben sich dabei lediglich in Hinblick auf die Kompaktierbarkeit des Pulvers und der Festigkeit der daraus erzeugten Formkörper. Einer Verminderung der Kompaktierbarkeit bzw. Festigkeit kann dabei jedoch vorteilhaft durch die Zugabe eines Bindemittels, bspw. Gelatine, Chitin, oder Chitosan, entgegengewirkt werden. Durch die Zugabe von pharmazeutischen Wirkstoffen können vorteilhaft Knochenersatzmaterialien erzeugt werden, die besonders gut vom Körper aufgenommen werden und den Heilungsprozess aktiv unterstützen. Dies kann bspw. durch Zugabe von entzündungshemmenden, sich in situ lösenden Wirkstoffen, wie z. B. Gentamicin, zum Kompositpulver erreicht werden.

Ein weiteres vorteilhaftes Merkmal besteht darin, dass die geometrischen Eigenschaften der Partikel des Kompositpulvers, insbesondere deren Äquivalentdurchmesser und/oder deren Form, durch die Substruktur der von dem Kollagen gebildeten selbstorganisierten Strukturen sowie den Zermahlvorgang bestimmt werden.

Vorteilhaft ist dabei vor allem der strukturelle Aufbau der einzelnen Pulverteilchen der durch Vermahlung der Xerogele erhaltenen Pulver. Diese entsprechen mikroskopisch kleinen Kompaktkompositen, die wiederum beim Verpressen durch Verzahnung, Kohäsion und chemische Bindung zu makroskopischen Kompaktkompositen zusammengefügt werden. Die geometrischen Eigenschaften der Partikel werden dabei zum einen durch die Substruktur der von dem Kollagen gebildeten selbstorganisierten Strukturen bestimmt. Eine Partikelgröße bzw. ein Äquivalentdurchmesser von etwa 10-50 µm, bevorzugt 5-50 µm und besonders bevorzugt von 1-50 µm kann daher auch bei wiederholtem Mahlvorgang nicht unterschritten werden, wenn die organische und die anorganische Phase in den Partikeln als Verbund vorliegen sollen. Zudem sind insbesondere die Partikelgröße (Äquivalentdurchmesser) und in begrenztem Umfang auch die Partikelform durch den Zermahlvorgang des Kompositmaterials bestimmt. So kann durch die Einstellung des Mahlgrades der verwendeten Mühle die Partikelgröße bereits innerhalb gewisser Grenzen eingestellt werden, bevor in einer quantitativen Siebanalyse eine genauere Auftrennung der Fraktionen erfolgt. Weiterhin können bevorzugt je nach verwendetem Mahlwerkzeug bzw. Mahlprozess Partikel gewonnen werden, die verschiedene Formen, bspw. plattenförmig, stangenförmig, nadelförmig oder kugelig, aufweisen. Werden diese Partikel anschließend zu einem Kompaktkomposit verpresst, wird dessen Struktur durch die Form der Partikel maßgeblich mitbestimmt. Dadurch kann zusätzlich eine Einstellung der Eigenschaften der Knochenersatzmaterialien bzw. eine exaktere Nachbildung verschiedener Knochentypen erfolgen.

In einer weiterhin bevorzugten Ausführungsform des erfindungsgemäßen Verfahrens werden das Kompositpulver und/oder das Kompaktkomposit chemisch und/oder physikalisch behandelt und dadurch das Kollagen und/oder die anorganische Phase vernetzt.

Dabei wird die Fähigkeit des Kollagens zur Vernetzung vorteilhaft für die Verbesserung der Kompaktierbarkeit und der Festigkeit des Kompaktkomposits genutzt. Dies ist möglich, da die Partikel des Kompositpulvers aus nur unregelmäßig mineralisiertem Kollagen bestehen, die organische Phase stellenweise also unverhüllt zu Tage tritt. Zur Vernetzung dieser Phase werden das Kompositpulver und/oder das Kompaktkomposit mit einer Vernetzungslösung, bspw. aus EDC (N-(3-Dimethylaminopropyl)-N'-ethylcarbodiimide hydrochloride) und NHS (N-Hydroxysuccinimide) behandelt. Dies führt vorteilhaft zur Ausbildung von Bindungen zwischen dem Kollagen verschiedener Partikel, wodurch eine weitere Verbesserung der mechanischen Eigenschaften des Kompaktkomposits erreicht wird. Weiterhin bevorzugt ist eine Vernetzung der anorganischen Phase durch Begasung des Kompositpulvers und/oder des Kompaktkomposits mit einem Vernetzungsgas, bspw. NH₃.

Die interfibrilläre Vernetzung des Kollagens kann weiterhin auch durch eine physikalische dehydrothermale Behandlung verstärkt und dadurch eine Verbesserung der mechanischen Eigenschaften des Kompaktkomposits bewirkt werden. Bei der dehydrothermalen Behandlung, werden das Kompaktkomposit und insbesondere das darin enthaltene Kollagen über längere Zeiträume und im Vakuum auf Temperaturen von bis zu 95°C erhitzt. Dies führt zur Entwässerung des Kollagens und befördert die Ausbildung intermolekularer Verbindungen zwischen den Seitenketten von Aminosäuren der Kollagenfasern durch Kondensationsreaktionen unter Veresterung oder Amidbildung. Die so erhöhte Quervernetzung der Kollagenfasern untereinander bewirkt insbesondere einen Anstieg des Kompressions- und Zugmoduls des die vernetzten Fasern enthaltenden Kompaktkomposits.

Die dehydrothermale Behandlung führt darüber hinaus jedoch auch zu einer Denaturierung des Kollagens, also zur Neuordnung der Dreifachhelix der Fasern in eine irregulär verkettete Konfiguration. Wie in der Vergangenheit gezeigt werden konnte [Haugh et al. The effect of dehydrothermal treatment on the mechanical and structural properties of collagen GAG scaffolds. Royal College of Surgeons in Ireland, Anatomy Articles 1-5-2009], steigt der Anteil der denaturierten Fasern mit der Dauer und der Temperatur der Behandlung an. Das auf diesem Wege denaturierte Kollagen trägt dabei jedoch unter Umständen stärker zur Geweberegeneration bei als die ursprünglichen Fasern [Koide et al. A new type of biomaterial for artificial skin: dehydrothermally cross-linked composites of fibrillar and denatured collagens. J Biomed Mater Res 1993,27(1):79-87]. Darüber hinaus ist denaturiertes Kollagen zu einer schnelleren Degradation fähig als nicht denaturiertes. Die biologische Aktivität der in dem Kompaktkomposit enthaltenen Fasern wird somit durch die dehydrothermale Behandlung nach dem Verpressen des Kompositpulvers nicht eingeschränkt.

Weiterhin Gegenstand der Erfindung ist ein homogenisiertes Kompaktkomposit, das durch ein vorab beschriebenes erfindungsgemäßes Verfahren erhältlich ist und ein defektarmes Gefüge, aus ineinander verzahnten und/oder chemisch und/oder durch Nebenvalenzbindungen gebundenen Partikeln mit einer definierte Partikelgrößenverteilung und einen Äquivalentdurchmesser, bevorzugt einen volumenäquivalenten Kugeldurchmesser, von weniger als 0,700 mm, bevorzugt zwischen 0,001 und 0,700 mm, besonders bevorzugt zwischen 0,05 und 0,700 mm und ebenfalls bevorzugt zwischen 0,250 und 0,700 mm, aus einzeln oder als Agglomerat vorliegendem, unregelmäßig mit Silikat, bevorzugt mit Silikat und einer Calciumphosphatphase, mineralisiertem Kollagen aufweist.

Wie oben beschrieben werden die Partikel dabei durch Zermahlen eines Kompositmaterials gewonnen. Zuvor innere Oberflächen des i.d.R. monolithischen Ausgangsmaterials, bspw. eines Xerogels, werden dadurch zu äußeren Oberflächen der Pulverpartikel. Dadurch weisen die Partikel vorzugsweise eine sehr große spezifische Oberfläche im Verhältnis zu Ihrem Volumen auf, was wiederum eine hohe Reaktivität der Teilchen bedingt. Diese Reaktivität zeigen dabei sowohl die an der Partikeloberfläche vorliegenden anorganischen Phasen, als auch das zum Teil an der Oberfläche der Partikel vorliegende Kollagen. Im homogenisierten Kompaktkomposit sind diese Partikel daher vorteilhaft chemisch, bspw. kovalent, oder durch Nebenvalenzbindung gebunden oder durch vielfach wechselseitigen geometrischen Formschluss der Partikel untereinander "verzahnt" und dadurch verbunden. Verzahnt bezieht sich somit auf eine rein mechanische, allein durch die Form der Partikel bedingte mikroskopische Fügung derselben zu einem makroskopisch homogenen Kompaktkomposit.

Dieses Kompaktkomposit wird nicht durch Trocknung eines Hydrogels sondern durch Kompaktierung eines Kompositpulvers gewonnen. Dadurch kommt es im Herstellungsprozess nicht zu einer Volumenkontraktion, die mit dem Auftreten von Defekten, insbesondere Rissen, und Spannungen im Material verbunden ist. Das erfindungsgemäße Kompaktkomposit weist daher ein homogenes und im Vergleich zu aus dem Stand der Technik bekannten Knochenersatzmaterialien defektarmes Gefüge auf.

Das Gefüge des erfindungsgemäßen Kompaktkomposits kann weiterhin auf vielfältige Art und Weise eingestellt werden. Es wird dabei maßgeblich durch die Form der Partikel des Kompositpulvers bestimmt, die beim Zermahlen des Kompositmaterials, bspw. durch die Wahl der Mühle einstellbar ist. So können Partikel gewonnen werden, die verschiedene Formen, bspw. plattenförmig, stangenförmig, nadelförmig oder kugelig, aufweisen. Werden diese Partikel anschließend zu einem Kompaktkomposit verpresst, wird dessen Gefüge durch die Form der Partikel maßgeblich mitbestimmt. Die Form und Größe der Partikel haben ebenfalls Einfluss auf Form und Größe der Hohlräume des Kompaktkomposits. Weiterhin kann die Textur des Kompkatkomposits, bspw. durch die Zugabe eines Bindemittels, beeinflusst werden.

Das erfindungsgemäße Kompaktkomposit ist mesoporös und weist eine Porenverteilung im Bereich von 2 bis 50 nm auf.

Bevorzugt ist das homogenisierte Kompaktkomposit beliebig geometrisch umformbar sowie mechanisch beliebig bearbeitbar.

Wie bereits ausgeführt, wirken sich die Beschränkungen des Volumens homogen herstellbarer Hydrogele nicht auf das Kompaktkomposit aus, da dieses chargenübergreifend aus verschiedenen Pulveransätzen gepresst werden kann. Dadurch kann das Kompaktkomposit vorteilhaft in nahezu beliebiger, nur durch die verwendeten Presswerkzeuge limitierter Größe hergestellt werden. Weiterhin kann das Kompaktkomposit in Abhängigkeit der verwendeten Werkzeuge eine nahezu beliebige geometrische Form aufweisen. Bevorzugt ist zudem die Herstellung des Kompaktkomposits in mehrstufigen Pressverfahren, wodurch die Gestaltungsfreiheit hinsichtlich der Form des Komposits weiter erhöht wird. Das Kompaktkomposit ist vorteilhaft beliebig urformbar und lässt sich als Knochenersatzmaterial zu Implantaten oder Scaffolds nahezu beliebiger Geometrie verarbeiten.

Da das Gefüge des Kompaktkomposits weniger Defekte und Spannungen aufweist als die durch Trocknung von Hydrogelen gewonnen Kompositmaterialien, ist es zudem auch wesentlich besser bearbeitbar. Aus dem mechanisch beliebig bearbeitbarem Kompaktkomposit können daher vorteilhaft auch komplexere Formen, die in Pressvorgängen oder bei der Trocknung von Hydrogelen nicht oder nur schwer realisierbar sind, erzeugt werden.

Das homogenisierte Kompaktkomposit weist bevorzugt eine scheinbare Dichte von 0,5-5 g/cm³, bevorzugt 1-3 g/cm³ und besonders bevorzugt 1,5-2,5 g/cm³ auf.

Die Dichte des homogenisierten Kompaktkomposits kann während des erfindungsgemäßen Verfahrens gezielt eingestellt werden. Dies erfolgt bevorzugt mechanisch durch die Einstellung des Drucks während des Verpressens des Kompositpulvers und/oder chemisch durch Porosierung. Die gezielte Einstellung der Dichte erlaubt vorteilhaft, Kompaktkomposite als vielfältige Knochenersatzmaterialien für verschiedene Knochentypen bereit zu stellen. Wird das Hohlraumvolumen des Materials nach dem Verpressen durch Porosierung erhöht, kann die Reindichte des Materials die scheinbare Dichte deutlich übersteigen.

Die Dichteverteilung des erfindungsgemäßen Kompaktkomposits ist dabei bevorzugt sowohl mikroskopisch als auch makroskopisch homogen. Dies stellt einen Vorteil gegenüber den bekannten in Sol-Gel-Verfahren hergestellten Kompositmaterialien dar, da bei diesen durch den Trocknungsprozess in der Regel Dichtegradienten im Material auftreten. Ist ein Dichtegradient im Kompaktkomposit jedoch erwünscht, bspw. zur biomimetischen Nachahmung eines bestimmen Knochentyps, kann er während des erfindungsgemäßen Verfahrens eingestellt werden. Dies kann entweder durch mehrstufige Pressverfahren oder durch Verwendung von Pulvermaterialien aus verschiedenen Pulveransätzen, insbesondere mit unterschiedlichen Partikelgrößenverteilungen, erfolgen.

Weiterhin bevorzugt weist das homogenisierte Kompaktkomposit eine biaxiale Festigkeit von über 30 MPa, bevorzugt zwischen 30 und 200 MPa und besonders bevorzugt zwischen 50 und 150 MPa auf.

Durch die Einstellung des Pressdrucks beim Kompaktieren kann die biaxiale Festigkeit, bevorzugt auch die Spaltzugfestigkeit, des Kompaktkomposits auch ohne die Zugabe von zusätzlichem Bindemittel gezielt eingestellt werden. Die biaxiale Festigkeit kann dabei mittels der "punch on three balls" Methode und die Spaltzugfestigkeit mittels der "3 ball on 3 ball" Methode *[vgl*. *"3-Balls-on-3-Balls Test for Ceramic Discs: A finite Element Study", T. Fett, G. Rizzi, Wissenschaftliche Berichte des Forschungszentrum Karlsruhe in der Helmholtz-Gesellschaft FZKA 7052] oder* gemäß der DIN EN 12390-6 im sogenannten "Brasilianertest" bestimmt werden.

Die mechanischen Eigenschaften des Kompaktkomposits hängen zusätzlich von den Eigenschaften des Kollagen und der anorganischen Phase ab. Prinzipiell sind alle anorganischen Phasen, insbesondere Silikate, bevorzugt Silikate und Calciumphosphatphasen, verwendbar die in einer homogenen Kollagensuspension löslich sind.

Als Kollagenkomponente des erfindungsgemäßen Kompaktkomposits kann jegliches Kollagen eingesetzt werden, d.h. Kollagen jeglicher Form (z. B. fibrilläres Kollagen, netzbildendes Kollagen, faserbildendes Kollagen, Tropokollagen, teilweise oder vollständig fibrilliertes Kollagen, teilweise oder vollständig (irreversibel) denaturiertes Kollagen, Kollagenfasern oder größere Aggregate usw.) und jeglichen Typs (z. B. Typ I, II, III, IV, V, VI, VII, VIII, IX, X, XI, XII, XIII, XIV, XV, XVI, XVII, XVIII, XIX, XX, XXI, XXII, XXIII, XXIV, XXV, XXVI).

Die Kollagenkomponente des erfindungsgemäßen Kompaktkomposits kann dabei rekombinantes Kollagen, Kollagen aus Eumetazoa (d.h. Gewebetiere, darunter Nesseltiere und Bilateria), Schwammkollagen der Klassen Demospongia (Hornschwämme) oder Calcarea (Kalkschwämme), ein synthetisches Kollagenanalogon, ein Kollagenderivat oder eine Mischung dieser Kollagene sein.

Kollagenanaloga sind synthetisch hergestellte Polypeptidketten, deren Primärsequenz so gestaltet ist, dass sie bestimmte Eigenschaften der nativen Kollagenmonomere simulieren können, z. B. Tripelhelixbildung, Fibrillogenese, Gelbildung. Rekombinante Kollagene sind solche, deren Primärsequenz identisch ist mit der von Kollagen Typ I. Sie werden mithilfe genetisch manipulierter Mikroorganismen hergestellt und gegebenenfalls nachbehandelt. Kollagenderivate sind Verbindungen, die sich von der Grundverbindung Kollagen formal ableiten und aus ihr herstellen lassen.

In einer besonders bevorzugten Ausführungsform wird die organische Komponente Kollagen zusätzlich biochemisch modifiziert. Die Modifikation der Kollagenkomponente beruht dabei auf einer gezielten Beeinflussung der Fibrillengeometrie (Durchmesser und Länge) durch biochemische Eingriffe in die Fibrillogenese. Da die Fibrillen im Kompositpulver und im Kompaktkomposit die Funktion einer Faserverstärkung einnehmen, hat ihre Geometrie Einfluss auf die mechanischen Eigenschaften der Verbundmaterialien.

Mithilfe von Aminozucker-haltigen Polysacchariden, bevorzugt von Glykosaminoglykanen und deren Analoga, lässt sich die Fibrillengeometrie vorteilhaft gezielt beeinflussen. Zu Glykosaminoglykanen analoge Verbindungen sind chemische Verbindungen mit gleicher biologischer Wirkung.

Besonders bevorzugt ist dabei die biochemische Modifikation des Kollagens durch die Glykosaminoglykane Chondroitinsulfat, Dermatansulfat, Heparin, Heparansulfat, Hyaluronsäure und Keratansulfat, aber auch durch Proteoglykane, wie Decorin, und andere Aminozucker-haltige Polysaccharide wie Chitosan. [Wolf C., Hanke T., Scharnweber D., Peters K., Kirkpatrick C. J., Worch H.: Influence of artificial extracellular matrices on Ti6AI4V implants on binding and release of VEGF, Abstracts Internationales Symposium Interface Biology of Implants', Rostock 14.-16.05.2003, in: BIOmaterialien 4: 158, 2003; Wolf-Brandstetter C., Lode A., Hanke T., Scharnweber D., Worch H.: "Influence of Modified Extracellular Matrices on Ti6AI4V implants on binding and release of VEGF". J. Biomed. Mat. Res A. 79: 882-894, 2006; Abschlussbericht "Innovative Materialsysteme für die Funktionalisierung von Implantaten und den Gewebeaufbau in der Implantologie" (Autoren: Worch, H., Scharnweber, D., Hanke, T., 2002) BMBF-Projekt 03N4015/9 (10/1999 bis 10/2002); Sammlung "Deutsche Forschungsberichte" TIB/UB Hannover].

Die biochemische Modifikation erfolgt durch den Einbau der genannten Substanzen in die Kollagenmatrix. Der Einbau erfolgt bevorzugt entweder durch adsorptive Immobilisierung, oder durch kovalente Quervernetzung, beispielsweise mit N-(3-dimethylaminopropyl)-N'-ethylcarbodiimide (EDC). Der Einbau kann alternativ auch während der spontanen Zusammenlagerung der Fibrillen stattfinden, die bei neutralem pH-Wert (z. B. in Phosphatpuffer oder Tris-Puffer) und auf ca. 37°C erhöhter Temperatur erreicht wird. Im Anschluss daran erfolgt dann gegebenenfalls eine kovalente Quervernetzung.

Die organische Komponente des erfindungsgemäßen Kompaktkomposits können somit auch vorstrukturierte Kollagentemplate, vorstrukturierte denaturierte Kollagene, vorstrukturierte Kollagenmoleküle, vorstrukturierte Kollagenmikrofibrillen, vorstrukturierte Kollagenanaloga und/oder vorstrukturierte Kollagenfragmente mit einer Länge von vorzugsweise 50 bis 500 nm sein. Dabei bezeichnet Vorstrukturierung im Sinne der Erfindung, dass die vorstrukturierten Kollagentemplate biochemisch modifiziert bzw. so funktionalisiert sind, dass an deren Oberfläche Hydroxylapatit aufwachsen kann, der aufgrund der Größe, Vorstrukturierung und Diffusions- und Migrationsfähigkeit der vorstrukturierten Kollagentemplate wachstumsinhibiert wird. Die Funktionalisierung kann natürlichen Ursprungs sein oder durch Vorstrukturierung mit Zuckermolekülen oder nichtkollagenen Proteinen erreicht werden. Für eine detaillierte Beschreibung der Erzeugung und Wirkung wachstumsinhibierten Hydroxylapatits wird auf die DE 10 2011 052 416 A1 verwiesen.

Die anorganische Komponente des erfindungsgemäßen Kompaktkomposits sind bevorzugt Silikate und/oder Calciumphosphatphasen.

Bei den Silikaten handelt es sich vorzugsweise um Sol-Gel-Silikate, hergestellt aus Silanen die zunächst hydrolisiert werden. Als Zwischenprodukt entsteht dabei Kieselsäure, die wiederum zu Silikat polymerisiert.

Der neben Kollagen zweite Hauptbestandteil von Knochen ist Calciumphosphat in der Form von Hydroxylapatit. Durch die Einführung von Hydroxylapatit und anderen Calciumphosphatphasen in das erfindungsgemäße Kompaktkomposit als zusätzliche anorganische Komponente, die nass-chemisch oder in pulvriger Form physikalisch in das System eingebracht wird, erhöhen sich der Feststoffanteil und die Bioaktivität des erfindungsgemäßen Kompaktkomposits ganz erheblich. Dies kann beispielsweise demonstriert werden durch die Beschleunigung der Ausbildung einer Hydroxylapatitschicht in simulierter Körperflüssigkeit (Simulated body fluid, SBF) *in vitro.*

Einsetzbar sind alle Calciumphosphatverbindungen, die eine homogene Mischung mit der Silikatlösung und der Kollagensuspension erlauben. Vorzugsweise finden dabei solche Calciumphosphatphasen Verwendung, die in *vivo* in Hydroxylapatit umgewandelt werden. In einer bevorzugten Ausführungsform wird als Calciumphosphatphase Hydroxylapatit (Ca₁₀(PO₄)₆(OH)₂), Tricalciumphosphat (Ca₃PO₄), amorphes Calciumphosphat (Caₓ(PO₄)_{y}·nH₂O), Octacalciumphosphat (Ca₈H₂(PO₄)₆·5H₂O) oder Brushit (CaHPO₄·2H₂O) oder Mischungen dieser Calciumphosphatphasen untereinander, wie beispielsweise Calciumphosphatzement (eine Mischung aus Ca₃(PO₄)₂, CaCO₃ Ca(HCO₃) und Ca₁₀(PO₄)₆(OH)₂), oder Mischungen von bzw. mit Calcium- und Phosphatsalzen wie beispielsweise CaCl₂ und/oder Na₂HPO₄ genutzt. Die Calciumphosphatphase kann dabei zusätzlich mit Ionen wie Fluorid, Silber, Magnesium oder Carbonat dotiert sein.

In einer besonders bevorzugten Ausführungsform wird als Calciumphosphatphase nanoskopischer Hydroxylapatit (Ca₁₀(PO₄)₆(OH)₂) eingesetzt, da sich dieser besonders homogen einbringen lässt. Eine homogene Verteilung der Calciumphosphatphase im Material bewirkt besonders gut reproduzierbare Materialeigenschaften. Nanoskopischer Hydroxylapatit besteht im Wesentlichen aus Hydroxylapatit- Nanopartikeln, die vorzugsweise eine Größe von 10 bis 500 nm, bevorzugt eine Größe von weniger als 100 nm aufweisen.

Nach einer weiteren bevorzugten Ausführungsform wird als Calciumphosphatphase wachstumsinhibierter Hydroxylapatit eingesetzt, wie er in DE 10 2011 052 416 A1 offenbart ist. Der wachstumsinhibierte Hydroxylapatit ist in Agglomeraten von vorstrukturierten Kollagentemplaten enthalten, an denen epitaktisch Hydroxylapatitkristallite mit einer Kristallitgröße unterhalb ihres kritischen Keimradius formiert sind. Hergestellt wird er mit den Schritten
a) Mineralisation von vorstrukturierten Kollagentemplaten in übersättigten Ca- und Phosphationen aufweisenden Lösungen, wobei die vorstrukturierten Kollagentemplate diffusions- bzw. migrationsfähig sind, so dass eptaktisch HAP-Kristallite an den Kollagentemplaten aufwachsen und sich die mit HAP-Kristalliten aufgewachsenen Kollagentemplate aufgrund deren Diffusions- bzw. Migrationsfähigkeit agglomerieren,
b) Abtrennen der Agglomerate.

Bei Verwendung von wachstumsinhibiertem Hydroxylapatit können vorteilhaft eine erhöhte Degradabilität des Implantats sowie eine vermehrte *in situ* Freisetzung von Calcium- und Phosphationen erreicht werden. Beides wirkt sich positiv auf die Einheilung des Implantats sowie die Umwandlung des Implantatmaterials in gesundes Gewebe um.

Bevorzugt weist das homogenisierte Kompaktkomposit eine Zusammensetzung von 10-95 Masse-% Silikat, 5-75 Masse-% Kollagen, bevorzugt 20-40 Masse-% Kollagen, und bis zu 60 Masse-% Calciumphosphat, bevorzugt bis zu 20 Masse-% Calciumphosphat auf.

Ebenfalls bevorzugt ist, dass das homogene Kompaktkomposit Additive wie pharmazeutische Wirkstoffgruppen und/oder Bindemittel enthält.

Diese können bereits nass-chemisch in das Ausgangskompositmaterial oder bei der Verpressung mit in das Kompaktkomposit integriert werden. Die Additive können dabei weniger als 5m-% bevorzugt weniger als 1m-% und besonders bevorzugt weniger als 0,1 m-% des Kompositpulvers (vor dem Verpressen) und somit des Kompaktkomposits ausmachen. Sollten sich bei hohen Additivkonzentrationen die Kompaktierbarkeit des Pulvers bzw. die mechanischen Eigenschaften des Presslings verschlechtern, kann dem vorteilhaft durch die zusätzliche Beimischung von Bindemitteln entgegengewirkt werden.

Weiterhin Gegenstand der Erfindung ist ein Kompositpulver mit Pulverpartikeln mit einer definierten Partikelgrößenverteilung und einem Äquivalentdurchmesser, bevorzugt einen volumenäquivalenten Kugeldurchmesser, von weniger als 0,700 mm, bevorzugt zwischen 0,001 und 0,700 mm, besonders bevorzugt zwischen 0,05 und 0,700 mm und ebenfalls bevorzugt zwischen 0,250 und 0,700 mm, aus einzeln oder als Agglomerat vorliegendem, unregelmäßig mit Silikat, mineralisiertem Kollagen, und einer Schüttdichte größer 0,1 g/cm³, bevorzugt zwischen 0,2 und 0,4 g/cm³ und besonders bevorzugt größer 0,5 g/cm³.

Gegenstand der Erfindung ist weiterhin ein Kompositpulver erhältlich durch Zermahlen eines Kompositmaterials aus einer mit Silikat mineralisierten Kollagenmatrix zu einem Kompositpulver, enthaltend Partikel aus einzeln oder als Agglomerat vorliegendem, unregelmäßig mit Silikat mineralisiertem Kollagen mit einem Äquivalentdurchmesser kleiner 0,700 mm. Das erfindungsgemäße, kompaktierbare Kompaktkomposit ist weiterhin dadurch gekennzeichnet, dass es bei Raumtemperatur und Pressdrücken von unter 1 GPa, bevorzugt zwischen 700 MPa und 1 GPa sowie ohne den Einsatz von zusätzlichen Bindemitteln zu einem formstabilen homogenisierten Kompaktkomposit mit einer biaxialen Festigkeit von 30 MPa bis 200 MPa, bevorzugt zwischen 50 und 150 MPa, verpressbar ist.

Besonders bevorzugt sind die oben beschriebenen Kompositpulver, enthaltend Pulverpartikel aus einzelnem oder als Agglomerat vorliegendem, unregelmäßig mit Silikat und einer Calciumphosphatphase mineralisiertem Kollagen.

Die Partikelgrößenverteilung kann dabei während des Zermahlvorgangs des Kompositmaterials gezielt eingestellt werden. Zusätzlich kann die Breite der Verteilung durch nachträgliche Selektionsverfahren, bspw. durch Siebung, weiter eingeschränkt werden. Ebenso denkbar ist eine Einstellung bzw. Selektion der Partikel hinsichtlich der Partikelform während oder nach dem Zermahlvorgang.

Das Pulvermaterial stellt ein Zwischenprodukt des erfindungsgemäßen Verfahrens dar, dass im weiteren Verlauf desselben zu Formkörpern verpresst wird. Wie oben beschrieben können dem Pulvermaterial dabei zuvor vorteilhaft hohe Konzentrationen von Additiven und/oder nichtlöslichen Additiven beigemischt werden. Dadurch können Kompaktkomposite mit einer Zusammensetzung erzeugt werden, die in nass-chemischen Verfahren nicht realisierbar sind.

Das Kompositpulver weist somit nach dem Zermahlen eine Zusammensetzung auf, die dem des Ausgangskompositmaterials entspricht. Bevorzugt ist dafür eine Zusammensetzung des Kompositpulvers nach dem Zermahlen von 40-92,5m-% Silikat, 7,5-60m-% Kollagen und bis zu 52,5m-% Calciumphosphat. Weiterhin bevorzugt sind Zusammensetzungen von 40-92,5m-% Silikat, 7,5-30m-% Kollagen und bis zu 30m-% Calciumphosphat. Besonders bevorzugt sind Zusammensetzungen von 60-80m-% Silikat, 10-20m-% Kollagen und bis zu 10-20m-% Calciumphosphat.

Vor dem Verpressen des Kompositpulvers zum Kompaktkomposit kann durch die physikalische Beimengung von anderen Pulveransätzen und/oder einzeln vorliegenden Substituenten des Ausgangskompositmaterials und/oder eines pharmazeutischen Wirkstoffs und/oder eines Bindemittels und/oder wachstumsinhibierten Hydroxylapatits die Zusammensetzung des Kompositpulvers geändert werden.

Das Kompositpulver weist somit vor dem Verpressen eine Zusammensetzung von 10-95 m-% Silikat, 5-75 m-% Kollagen, bevorzugt 20-40 m-% Kollagen, und bis zu 60 m-% Calciumphosphat, bevorzugt bis zu 20 m-% Calciumphosphat auf. Der Anteil zusätzlicher Additive, wie Bindemittel, pharmazeutische Wirkstoffe oder wachstumsinhibierter Hydroxylapatit, machen dabei weniger als 5m-%, bevorzugt weniger als 1m-% und besonders bevorzugt weniger als 0,1m% des Kompositpulvers aus.

Weiterhin Gegenstand der Erfindung ist die Verwendung eines kompaktierbaren Kompositpulvers zur Herstellung eines homogenisierten Kompaktkomposits.

Ebenso Gegenstand der Erfindung ist die Verwendung des erfindungsgemäßen homogenisierten Kompaktkomposits und des erfindungsgemäßen kompaktierbaren Kompositpulvers zur Füllung bzw. Reparatur von Knochendefekten in situ.

Ebenso Gegenstand der Erfindung ist ein erfindungsgemäßes Kompaktkomposit zur Verwendung als Implantatmaterial. Das erfindungsgemäße Kompaktkomposit kann dabei in beliebiger Form hergestellt und vorteilhaft im Bereich größerer Knochenschädigungen eingesetzt werden. Dadurch können Knochendefekte behandelt werden, deren Dimensionierung den Einsatz herkömmlicher Knochenersatzmaterialien ausschließt. Weiterhin vorteilhaft können diesen Kompaktkompositen pharmazeutisch aktive Substanzen zugesetzt werden, um den Heilungsprozess zu beschleunigen.

Ebenso Gegenstand der Erfindung ist ein erfindungsgemäßes Kompositpulver zur Verwendung als Implantatmaterial. Das Kompositpulver eignet sich dabei besonders als Implantatmaterial für eine in situ Anwendung im Bereich des Knochendefekts. Vorteilhaft ist zudem die Beimischung pharmazeutisch aktiver Substanzen, welche den Heilungsprozess unterstützen. Dies können beispielsweise Wachstumsfaktoren, Zytokine oder aber auch entzündungshemmende Substanzen oder Antibiotika sein.

Das Pulvermaterial selbst kann somit als Implantat- bzw. Knochenersatzmaterial verwendet werden. Das Pulver wird dabei entweder in Knochenrisse oder kleine Defekte so eingepresst, dass eine Verfestigung zu einem Kompaktkomposit in situ erfolgt. Weiterhin bevorzugt ist die Einbringung des Pulvermaterials in loser oder nur gering verfestigter Form, mit dem Ziel der schnellen Auflösung bzw. Degradation. Dies dient bspw. der gezielten Versorgung umliegender Knochen mit für den Knochenstoffwechsel notwendigen Substanzen.

Weiterhin Gegenstand der Erfindung ist auch ein therapeutisches Verfahren zur Behandlung von Knochendefekten mit einem erfindungsgemäßen Kompositpulver oder Kompaktkomposit umfassend die Schritte:
- Erzeugen eines Hydrogels durch Mischen eines Silizium-Precursors und einer Kollagensuspension,
- Trocknung des Hydrogels zu einem Kompositmaterial aus einer mit Silikat mineralisierten Kollagenmatrix in Form eines Xero- oder Aerogels,
- Zermahlen des Kompositmaterials zu einem Kompositpulver, enthaltend Partikel mit einem Äquivalentdurchmesser kleiner 0,700 mm, durch Zermahlen und unter Erhaltung des Verbunds zwischen Kollagen und anorganischer Phase,
- In situ-Applikation des Kompositpulver in einen Knochendefekt und Verpressen des Kompositpulvers zu einem Kompaktkomposit bei Temperaturen unter 40°C und Pressdrücken von 700 MPa bis 1 GPa oder

Urformen des Kompositpulvers zu einem formstabilen homogenisierten Kompaktkomposit mit defektarmen Gefüge durch Verpressen des Kompositpulvers bei Temperaturen unter 40°C und Pressdrücken von 700 MPa bis 1 GPa und anschließender in situ Applikation in den Knochendefekt. Vorteilhafterweise kann das Kompositpulver bzw. Kompaktkomposit Beimischungen pharmazeutisch aktiver Substanzen und/oder Bindemittel aufweisen.

Im Folgenden wird die Erfindung anhand einiger Ausführungsbeispielen erläutert, ohne sie auf diese einzuschränken.

### Ausführungsbeispiel 1:

Lyophilisat von Kalbshautkollagen (GfN GmbH, Wald-Michelbach) wird in neutralem Tris/HCl-Puffer (100 mM, pH 7,4) über 24 Stunden unter Rühren (Magnetrührer Heidolph MR Hei-End, 500 rpm) suspendiert, so dass sich eine Konzentration von 30 mg/ml ergibt. Die entsprechenden Volumina an Kieselsäure und Kollagensuspension richten sich nach der angestrebten Zusammensetzung. In diesem Ausführungsbeispiel wird eine Masse-Zusammensetzung von 70 % Silikat und 30 % Kollagen angeführt. Zur Erzeugung von Kieselsäure wird Tetraethoxysilan (TEOS, 99 % Sigma) durch Zugabe von Wasser und Salzsäure (10 mM) als Katalysator 24 Stunden bei 4°C hydrolysiert. Das molare Verhältnis von Tetraethoxysilan zu Wasser beträgt 1:4. Diese Lösung dient als Silikatkomponente und wird der Kollagensuspension zugeführt und intensiv vermischt. Nach wenigen Minuten bildet sich ein Hydrogel.

Das eingesetzte Volumen pro Probe und die Form, in die die Mischungen gegeben werden, bestimmen zunächst die Geometrie des Hydrogels und schlussendlich die erhaltene Geometrie des Xerogels. Ein Volumen von 500 µl in zylindrischen Gefäßen von 14 mm Durchmesser resultiert in scheibenförmigen Xerogelen von 5 mm Durchmesser und 2 mm Höhe. Ein Volumen von 3500 µl bei 15 mm Durchmesser resultiert in zylindrischen Xerogelen mit etwa 11 mm Durchmesser und 16 mm Höhe. Die Hydrogele werden unter Luftabschluss für 72 Stunden bei Raumtemperatur stabilisiert. Die Überführung der Hydrogele in Xerogele erfolgt bei geöffneten Gefäßen bei 37°C und 95 % relativer Feuchte (Klimakammer Espec SH-221). Nach 7 Tagen wird das Klima kontinuierlich über 24 Stunden auf 20°C und 10 % relative Feuchte reguliert.

Die erhaltenen Kompositxerogele werden mittels Kugelmühle (Precellys 24, Peqlab GmbH) zu Pulvern zermahlen. Mittels Mikrosiebset (Bel-Art Sienceware) wird das Pulver nach seiner Partikelgröße in Fraktionen aufgeteilt. Für die Weiterverarbeitung wird die Fraktion mit Partikelgrößen < 0,35 mm verwendet.

200 mg des erhaltenen Kompositpulvers werden in ein zylindrisches Presswerkzeug (KBr Die, ALFA AESAR, Durchmesser 13 mm) mit planparallelen Stempeln gegeben und mit einer Hydraulikpresse (Perkin Elmer) bei einer Presskraft von 100 kN und einem Pressdruck von ca. 750 MPa verpresst. Die erhaltenen scheibenförmigen Proben haben eine Dicke von 1 mm.

Diese Proben werden beispielsweise genutzt, um die mechanischen Eigenschaften des Materials zu charakterisieren. Dazu werden diese im *ball on* 3 *balls-Test* belastet und Kraft/Verformungs-Kurven aufgezeichnet.

Die erhaltenen Kompaktkomposite sind nassfest. Die organischen Phasen des Kompaktkomposits werden chemisch vernetzt. Dies geschieht, indem 100mM EDC und 50mM NHS in 40% Ethanol gelöst und für 24 Stunden auf den Proben belassen werden. Anschließend wird die Vernetzungslösung abgesaugt und die Proben fünfmal je 30 min mit entionisiertem Wasser gespült. Die anorganisch-nichtmetallische Silikatphase wird durch Begasung in einem Exsikkator für 48 Stunden mittels NH₃ vernetzt.

### Ausführungsbeispiel 2:

Wie Ausführungsbeispiel 1, wobei zusätzlich Hydroxylapatit (Ca₁₀(PO₄)₆(OH)₂) (Merck, Artikelnummer 102143) in Pulverform in die Kollagensuspension gegeben und über 24 Stunden unter Rühren gelöst wird. Die einzuwiegende Menge an Calciumphosphatphasen richtet sich nach der angestrebten Zusammensetzung. Im Folgenden wird die mit Calciumphosphatphasen angereicherte Kollagensuspension wie in Ausführungsbeispiel 1 mit Kieselsäure gemischt und weiterverarbeitet. Eine vorteilhafte Masse-Zusammensetzung ist 70 % Silikat, 15 % Kollagen und 15 % Calciumphosphatphase.

### Ausführungsbeispiel 3:

Wie Ausführungsbeispiel 1. Die Presswerkzeuge wurden so konstruiert und gefertigt, dass bei Verpressen der Pulver bereits die endgültige Implantatgeometrie vorliegt. Die dazu notwendigen Patientendaten können beispielsweise mittels MRT-Scans ermittelt und in Maschinendaten umgerechnet werden.

### Ausführungsbeispiel 4:

Wie Ausführungsbeispiel 1. Die Presswerkzeuge wurden so konstruiert und gefertigt, dass bei Verpressen der Pulver Rohlinge entstehen, die zur Fixierung mittels handelsüblichen Spannwerkszeugen in Maschinen zur spanabhebenden Bearbeitung geeignet sind. Auf diesem Wege werden patientenspezifische Implantate gefertigt. Die dazu notwendigen Patientendaten können beispielsweise mittels MRT-Scans ermittelt und in Maschinendaten umgerechnet werden.

### Ausführungsbeispiel 5:

Ausgewählte Gelatine wird in geeigneten calcium- und phosphathaltigen Elektrolytlösungen mineralisiert. Dabei entstehen Komposite bestehend aus Gelatine im Kern und Hydroxylapatit auf der Oberfläche. Diese Komposite sind in der Regel selbstassemblierend und formen Kugeln unterschiedlicher Größe. Diese Kugeln werden in der beschriebenen Weise kompaktiert und daraus Festkörper hergestellt.

## Patentansprüche

1. Verfahren zur Herstellung eines homogenisierten Kompaktkomposits aus unregelmäßig silikatisiertem Kollagen, **aufweisend** die Verfahrensschritte
a) Erzeugen eines Hydrogels durch Mischen eines Silizium-Precursors und einer Kollagensuspension,
b) Trocknung des Hydrogels zu einem Kompositmaterial aus einer mit Silikat mineralisierten Kollagenmatrix in Form eines Xero- oder Aerogels,
c) Zermahlen des Kompositmaterials zu einem Kompositpulver, enthaltend Partikel mit einem Äquivalentdurchmesser kleiner 0,700 mm, durch Zermahlen und unter Erhaltung des Verbunds zwischen Kollagen und anorganischer Phase,
d) Urformen eines formstabilen homogenisierten Kompaktkomposit mit defektarmen Gefüge durch Verpressen des Kompositpulvers bei Temperaturen unter 40°C und Pressdrücken von 700 MPa bis 1 GPa.

2. Verfahren nach Anspruch 1, **wobei** in Verfahrensschritt a) das Hydrogel durch Mischen eines Silizium-Precursors und einer Calciumphosphatphase mit einer Kollagensuspension erzeugt wird.

3. Verfahren nach einem der Ansprüche 1 oder 2, **dadurch gekennzeichnet,**
**dass** die scheinbare Dichte des homogenisierten Kompaktkomposits in Abhängigkeit des Pressdrucks von 0,5 bis 5 g*cm⁻³ eingestellt wird.

4. Verfahren nach einem der Ansprüche 1 bis 3, **dadurch gekennzeichnet,**
**dass** vor dem Verpressen Porogene zum Kompositpulver zugegeben und nach dem Verpressen aus dem Gefüge des Kompaktkomposits gelöst werden.

5. Verfahren nach einem der Ansprüche 1 bis 4, **dadurch gekennzeichnet,**
**dass** vor dem Verpressen dem Kompositpulver pharmazeutische Wirkstoffe und/oder ein Bindemittel zugesetzt werden.

6. Homogenisiertes Kompaktkomposit, **erhältlich durch** ein Verfahren nach einem der Ansprüche 1 bis 5,
a) aufweisend ein defektarmes Gefüge und eine biaxiale Festigkeit von über 30 MPa,
b) enthaltend formschlüssig verbundene und/oder chemisch und/oder durch Nebenvalenzbindungen gebundene Partikel, wobei die Partikel
i. einen Äquivalentdurchmesser kleiner 0,700 mm aufweisen und
ii. aus einzeln oder als Agglomerat vorliegendem, unregelmäßig mit Silikat mineralisiertem Kollagen ausgebildet sind.

7. Homogenisiertes Kompaktkomposit nach Anspruch 6, **dadurch gekennzeichnet dass** es eine scheinbare Dichte von 0,5 bis 5 g/cm³ aufweist.

8. Homogenisiertes Kompaktkomposit nach Anspruch 6 oder 7, **dadurch gekennzeichnet, dass** es sich bei dem Kollagen um rekombinantes Kollagen, Kollagen aus Eumetazoa, Schwammkollagen der Klassen Demospongia oder Calcarea, um synthetische Kollagenanalogon, ein Kollagenderivat oder eine Mischung dieser Kollagene handelt.

9. Homogenisiertes Kompaktkomposit nach einem der Ansprüche 6 bis 8, **dadurch gekennzeichnet, dass** das Kollagen durch ein Aminozucker-haltiges Poylsaccharid und/oder ein Proteoglykan oder Analoga davon oder durch Chondroitinsulfat, Dermatansulfat, Heparin, Heparansulfat, Decorin, Chitosan, Hyaluronsäure und/oder Keratinsulfat biochemisch modifiziert ist.

10. Homogenisiertes Kompaktkomposit nach einem der Ansprüche 6 bis 9, **dadurch gekennzeichnet, dass** die Calciumphosphatphase aus Hydroxylapatit (Ca₁₀(PO₄)₆(OH)₂), Tricalciumphosphat (Ca₃PO₄), amorphem Calciumphosphat (Caₓ(PO₄)_{y}·nH₂O), Octacalciumphosphat (Ca₈H₂(PO₄)₆·5H₂O), Brushit (CaHPO₄·2H₂O), wachstumsinhibiertem Hydroxylapatit, aus Mischungen dieser Calciumphosphatphasen untereinander oder mit Calcium- und Phosphatsalzen ausgewählt und in vivo in Hydroxylapatit umwandelbar ist.

11. Homogenisiertes Kompaktkomposit nach einem der Ansprüche 6 bis 10, **dadurch gekennzeichnet, dass** es einen pharmazeutischen Wirkstoff und/oder ein Bindemittel enthält.

12. Kompaktierbares Kompositpulver,
a) aufweisend eine Zusammensetzung aus 10-95 m-% Silikat, 5-75 m-% Kollagen, 0-60 m-% Calciumphosphat und 0-5 m-% zusätzlicher Additive und
b) enthaltend Partikel
i. mit einen Äquivalentdurchmesser kleiner 0,700 mm,
ii. ausgebildet aus, einzeln oder als Agglomerat vorliegendem, unregelmäßig mit Silikat mineralisiertem Kollagen.

13. Kompaktierbares Kompositpulver,
a) erhältlich durch Zermahlen eines Kompositmaterials aus einer mit Silikat oder einer mit Silikat und einer Calciumphosphatphase mineralisierten Kollagenmatrix zu einem Kompositpulver,
b) enthaltend Partikel
i. mit einem Äquivalentdurchmesser kleiner 0,700 mm
ii. ausgebildet aus, einzeln oder als Agglomerat vorliegendem, unregelmäßig mit Silikat mineralisiertem Kollagen.

14. Kompaktierbares Kompositpulver nach Anspruch 12 oder 13, **dadurch gekennzeichnet, dass** es bei Temperaturen unter 40°C, bei Pressdrücken von 700 MPa bis zu 1 GPa und ohne den Einsatz zusätzlicher Bindemittel zu einem formstabilen homogenisierten Kompaktkompositmit defektarmen Gefüge und einer biaxialen Festigkeit von 30 bis 200 MPa verpressbar ist.

15. Verwendung eines Kompaktkomposits nach einem der Ansprüche 6 bis 11 oder eines Kompositpulvers nach einem der Ansprüche 12 bis 14 als Implantatmaterial.

## Claims

1. A method for the production of a homogenized compact composite formed from irregular silicatized collagen, comprising the following steps of the method:
a) producing a hydrogel by mixing a silicon precursor and a collagen suspension,
b) drying the hydrogel to form a composite material formed from a collagen matrix mineralized with silicate in the form of a xero- or aerogel,
c) pulverizing the composite material into a composite powder containing particles with an equivalent diameter of less than 0.700 mm, by pulverizing with the production of the composite between the collagen and inorganic phase,
d) casting a homogenized compact composite which is stable in shape having a low-defect microstructure by pressing the composite powder at temperatures below 40°C and pressing forces of 700 MPa to 1 GPa.

2. The method as claimed in claim 1, wherein in method step a), the hydrogel is produced by mixing a silicon precursor and a calcium phosphate phase with a collagen suspension.

3. The method as claimed in claim 1 or claim 2, **characterized in that** the apparent density of the homogenized compact composite is adjusted to 0.5 to 5 g/cm³ as a function of the pressing force.

4. The method as claimed in one of claims 1 to 3, **characterized in that** prior to pressing, pore-forming agents are added to the composite powder and after pressing, they are dissolved out of the microstructure of the compact composite.

5. The method as claimed in one of claims 1 to 4, **characterized in that** prior to pressing, pharmaceutically active ingredients and/or a binder is/are added to the composite powder.

6. A homogenized compact composite obtainable by means of a method as claimed in one of claims 1 to 5,
a) having a low-defect microstructure and a biaxial strength of more than 30 MPa,
b) containing mechanically bonded and/or chemically bonded and/or weakly interacting particles, wherein the particles
i. have an equivalent diameter of less than 0.700 mm, and
ii. are formed from collagen which has been irregularly mineralized with silicate, individualized or in the form of agglomerates.

7. The homogenized compact composite as claimed in claim 6, **characterized in that** it has an apparent density of 0.5 to 5 g/cm³.

8. The homogenized compact composite as claimed in claim 6 or claim 7, **characterized in that** the collagen is recombinant collagen, collagen from Eumetazoa, sponge collagen from the Demospongia or Calcarea classes, synthetic collagen analogues, a collagen derivative or a mixture of said collagens.

9. The homogenized compact composite as claimed in one of claims 6 to 8, **characterized in that** the collagen is biochemically modified by an aminosugar-containing polysaccharide and/or a proteoglycan or analogues thereof or by chondroitin sulphate, dermatan sulphate, heparin, heparan sulphate, decorin, chitosan, hyaluronic acid and/or keratin sulphate.

10. The homogenized compact composite as claimed in one of claims 6 to 9, **characterized in that** the calcium phosphatase is selected from hydroxyapatite (Ca₁₀(PO₄)₆(OH)₂), tricalcium phosphate (Ca₃PO₄), amorphous calcium phosphate (Caₓ(PO₄)_{y}·nH₂O), octacalcium phosphate (Ca₈H₂(PO₄)₆·5H₂O), brushite (CaHPO₄·2H₂O), growth-inhibiting hydroxyapatite, and mixtures of these calcium phosphate phases either with each other or with calcium and phosphate salts, and can be transformed in vivo to form hydroxyapatite.

11. The homogenized compact composite as claimed in one of claims 6 to 10, **characterized in that** it contains a pharmaceutically active ingredient and/or a binder.

12. A compactable composite powder,
a) comprising a composition formed from 10-95% by weight of silicate, 5-75% by weight of collagen, 0-60% by weight of calcium phosphate and 0-5% by weight of additional additives, and
b) containing particles
i. with an equivalent diameter of less than 0.700 mm,
ii. formed from collagen irregularly mineralized with silicate, individualized or as an agglomerate,

13. A compactable composite powder,
a) which can be obtained by pulverizing a composite material formed from a collagen matrix mineralized with silicate or with a silicate and a calcium phosphate phase,
b) containing particles
i. with an equivalent diameter of less than 0.700 mm,
ii. formed from collagen irregularly mineralized with silicate, individualized or as an agglomerate.

14. The compactable composite powder as claimed in claim 12 or claim 13, **characterized in that** it can be pressed at temperatures of less than 40°C, at pressing forces of 700 MPa to 1 GPa and without using additional binders to form a homogenized compact composite which is stable in shape, with a defect-free microstructure and a biaxial strength of 30 to 200 MPa.

15. Use of a compact composite as claimed in one of claims 6 to 11 or of a composite powder as claimed in one of claims 12 to 14, as an implant material.

## Revendications

1. Procédé destiné à fabriquer un composite compact homogénéisé en collagène irrégulièrement silicaté, comportant les étapes de procédé
a) Création d'un hydrogel par mélange d'un précurseur de silicium et d'une suspension de collagène,
b) Séchage de l'hydrogel en une matière composite en une matrice de collagène minéralisée avec du silicate sous la forme d'un xérogel ou d'un aérogel,
c) Broyage de la matière composite en une poudre composite contenant des particules d'un diamètre équivalent inférieur à 0,700 mm, par broyage et sous obtention de la liaison entre le collagène et la phase anorganique,
d) Façonnage d'un composite compact homogénéisé indéformable avec des structures présentant peu de défauts par compression d'une poudre composite à des températures inférieures à 40 °C et des puissances de pression de 700 MPa à 1 GPa.

2. Procédé selon la revendication 1, dans lequel dans l'étape de procédé a), l'hydrogel est produit par mélange d'un précurseur de silicium et d'une phase de phosphate de calcium avec une suspension de collagène.

3. Procédé selon l'une quelconque des revendications 1 ou 2, **caractérisé en ce qu'**on règle la densité apparente du composite compact homogénéisé entre 0,5 et 5g*cm⁻³, en fonction de la puissance de pression.

4. Procédé selon l'une quelconque des revendications 1 à 3, **caractérisé en ce qu'**avant la compression, on ajoute à la poudre composite des agents porogènes et après la compression, on les dissout hors de la structure du composite compact.

5. Procédé selon l'une quelconque des revendications 1 à 4, **caractérisé en ce qu'**avant la compression, on ajoute à la poudre composite des principes actifs et/ou un agent liant.

6. Composite compact homogénéisé susceptible d'être obtenu par un procédé selon l'une quelconque des revendications 1 à 5,
a) comportant une structure présentant peu de défauts et une résistance biaxiale supérieure à 30 MPa,
b) contenant des particules reliées par complémentarité de forme et/ou liées chimiquement et/ou liées par des liaisons à valence secondaire, les particules
i. présentant un diamètre équivalent inférieur à 0,700 mm et
ii. étant conçues en tant que collagène minéralisé irrégulièrement avec du silicate, présent individuellement ou sous forme agglomérée.

7. Composite compact homogénéisé selon la revendication 6, **caractérisé en ce qu'**il présente une densité apparente de 0,5 à 5g/cm³.

8. Composite compact homogénéisé selon la revendication 6 ou la revendication 7, **caractérisé en ce que** le collagène est un collagène recombinant, un collagène provenant d'Eumetazoa, un collagène d'éponge provenant d'une éponge de la classe Desmopongia (éponge en corne) ou Calcarea (éponge calcaire), un analogue synthétique collagène, un dérivé de collagène ou un mélange desdits collagènes.

9. Composite compact homogénéisé selon l'une quelconque des revendications 6 à 8, **caractérisé en ce que** le collagène est biochimiquement modifié par un polysaccharide contenant un sucre aminé et/ou un protéoglycane ou un analogue de ces derniers, un sulfate de chondroïtine, un sulfate de dermatane, de l'héparine, un sulfate d'héparane, de le décorine, du chitosan, de l'acide hyaluronique et/ou du sulfate de kératine.

10. Composite compact homogénéisé selon l'une quelconque des revendications 6 à 9, **caractérisé en ce que** la phase de phosphate de calcium est choisie parmi l'hydroxyapatite (Ca₁₀(PO₄)₆(OH)₂), le phosphate de tricalcium (ca₃PO₄), le phosphate de calcium amorphe (Caₓ(PO₄)_{y}·nH₂O), le phosphate d'octacalcium (Ca₈H₂(PO₄)₆·5H₂O), la brushite, l'hydroxyapatite à croissance inhibée, parmi des mélanges desdites phases de phosphate de calcium entre elles ou avec des sels de calcium et de phosphate et est susceptible d'être transformée in-vivo en hydroxyapatite.

11. Composite compact homogénéisé selon l'une quelconque des revendications 6 à 10, **caractérisé en ce qu'**il contient un principe actif pharmaceutique et/ou un agent liant.

12. Poudre composite compactée,
a) présentant une composition de 10 à 95 % en moles de silicate, de 5 à 75 % en moles de collagène, de 0 à 60 % en moles de phosphate de calcium et de 0 à 5 % en moles d'additifs supplémentaires
b) contenant des particules
i. avec un diamètre équivalent inférieur à 0,700 mm,
ii. qui sont conçues en tant que collagène minéralisé avec du silicate, présent individuellement ou sous forme agglomérée.

13. Poudre composite compactée,
a) susceptible d'être obtenue par broyage d'une matière composite en une matrice de collagène minéralisée avec du silicate ou avec du silicate et une phase de phosphate de calcium en une poudre composite,
b) contenant des particules
i. avec un diamètre équivalent inférieur à 0,700 mm,
ii. qui sont conçues en tant que collagène minéralisé avec du silicate, présent individuellement ou sous forme agglomérée.

14. Poudre composite compactée selon la revendication 12 ou la revendication 13, **caractérisée en ce qu'**elle est compressible à des températures inférieures à 40 °C, à des puissances de pression de 700 MPa à 1 GPa et sans l'utilisation d'agents liants supplémentaires en un composite compact homogénéisé indéformable, avec une structure présentant peu de défauts et avec une résistance biaxiale de 30 à 200 MPa.

15. Utilisation d'un composite compact selon l'une quelconque des revendications 6 à 11 ou d'une poudre composite selon l'une quelconque des revendications 12 à 14 en tant que matière pour des implants.
